# EUROPÄISCHE PATENTSCHRIFT

(11) **EP 0 983 290 B1**
(45) Veröffentlichungstag und Bekanntmachung des Hinweises auf die Patenterteilung: **03.03.2004**
(21) Anmeldenummer: 98928268.6
(22) Anmeldetag: 14.05.1998
(51) Int. Cl.: C07H 21/00

(54) **VERFAHREN ZUR HERSTELLUNG VON POLYMEREN, DIE NUCLEOBASEN ALS SEITENGRUPPEN AUFWEISEN**
METHOD FOR PRODUCING POLYMERS HAVING NUCLEO-BASES AS SIDE-GROUPS
PROCEDE DE PRODUCTION DE POLYMERES PRESENTANT DES NUCLEOBASES COMME GROUPES LATERAUX

(30) Priorität: 14.05.1997 DE 19720216; 14.05.1997 DE 19720165
(43) Veröffentlichungstag der Anmeldung: 08.03.2000
(73) Patentinhaber: Morphochem AG, 82152 Martinsried (DE)
(72) Erfinder: DÖMLING, Alexander, D-81243 München (DE); RICHTER, Wolfgang, D-81243 München (DE)
(74) Vertreter: Forstmeyer, Dietmar, Dr. rer. nat., Dipl.-Chem.
(86) Internationale Anmeldenummer: PCT/EP1998/002860
(87) Internationale Veröffentlichungsnummer: WO 1998/051697

(56) Entgegenhaltungen:
- EP-A- 0 029 909
- EP-A- 0 672 661
- EP-A- 0 739 898
- WO-A-95/02566
- WO-A-96/15143
- DE-A- 19 532 553
- SHORT K M ET AL: "Exploitation of the Ugi 4CC Reaction: Preparation of Small Molecule Combinatorial Libraries via Solid Phase" TETRAHEDRON, Bd. 53, Nr. 19, 12. Mai 1997, Seite 6653-6679 XP004105657
- RICHTER L S ET AL: "Synthesis of peptide nucleic acids (PNA) by submonomer solid-phase synthesis" BIOORGANIC & MEDICINAL CHEMISTRY LETTERS, Bd. 5, Nr. 11, 8. Juni 1995, Seite 1159-1162 XP004135496
- IVAR UGI ET AL: "Ugi Reactions with Trifunctional alpha-Aminoacids, Aldehydes, Isocyanides and Alcohols" TETRAHEDRON, Bd. 52, Nr. 35, August 1996, Seiten 11657-11664, XP002084702
- BREIPOHL G ET AL: "Novel Synthetic Routes to PNA Monomers and PNA-DNA Linker Molecules" TETRAHEDRON, Bd. 53, Nr. 43, 27. Oktober 1997, Seite 14671-14686 XP004106298
- HOPPE I. ET AL LIEBIGS ANN.CHEM. 1981, Seiten 103 - 106
- YOSHIDA J. ET AL J.CHEM.SOC.CHEM.COMM. 1994, Seite 549
- GOLLNICK K. ET AL J.ORG.CHEM. Bd. 57, 1992, Seite 229
- VRETBLAD P. ET AL ACTA CHEM.SCAND. Bd. 27, 1973, Seite 2769
- HARTKE K. CHEM.BER. 1966, Seite 3163
- BALIEU E. ET AL ACTA CHEM. SCAND. Bd. 26, 1972, Seite 2951

## Beschreibung

Die Erfindung betrifft ein Verfahren zur Herstellung von Polymeren, die Nucleobasen als Seitengruppen aufweisen, mittels Multi-Komponenten Reaktionen, speziell der Ugi-Reaktion.

Ferner betrifft die vorliegende Erfindung multifunktionelle Isonitrile, Verfahren zu deren Herstellung und deren Verwendung in Multikomponentenreaktionen. Derartige Isonitrile können in dem erfindungsgemäßen Verfahren zur Herstellung von Nucleobasen als Seitengruppen aufweisenden Polymeren eingesetzt werden.

In der organischen Synthese stellen Multikomponentenreaktionen (MCRs) wertvolle Verfahren dar. Sie werden z.B. zum Aufbau von Antibiotika, Peptiden etc., d.h. komplexen Molekülen mit einem hohen Diversitätsgrad eingesetzt. Bei üblichen MCRs wie z.B. einer Vier-Komponentenreaktion (4-CC) werden ein Isonitril, ein Aldehyd, eine Carbonsäure und ein Amin zu einem definierten Produkt umgesetzt. Als Isonitrile kommen dabei bisher monofunktionelle Verbindungen zum Einsatz, so daß die Vielfalt der Produkte derartiger Mehrkomponentenreaktionen beschränkt ist (vgl. Isonitrile Chemistry; I. Ugi (Ed.), Academic Press, New York, London 1971) und die entsprechende Reaktion abgeschlossen ist, sobald die einzelnen Komponenten abreagiert haben.

Mittels polymeren peptidischen Nucleinsäuren (PNA) regulierend auf die Genexpression einzuwirken wurde erstmals in den 60er Jahren von Svachkin et al. beschrieben (R. A. Paégle, M. G. Plata, M. Yu. Lidak, S. A. Giller, Yu. P. Shvachkin, in: Present State of the Chemotherapy of Malignant Tumors [in russisch], Riga (1968), 103 ff; Review: Yu. P. Shavachkin, G. P. Mishin, G. A. Korshunova, Russian Chemical Reviews, 51, 1982, 178 - 188). 1978 haben Zamecnik und Stephenson die Begriffe Antisense und Antigen eingeführt: Diese Begriffe beschreiben Mechanismen, wie therapeutisch in die Translation und Transkription von Genen eingegriffen werden kann (Proc. Natl. Acad. Sci. U.S.A., 1978, 75, 280 und 285).

Bei der Antisense Strategie bindet ein Antisense Molekül an die mRNA und verhindert somit deren Translation zu einem Protein. Bei der Antigen Strategie wird eine Tripelhelix des Antigen Moleküls mit der doppelsträngigen DNA gebildet und somit die Transkription in die mRNA modifiziert (E. Uhlmann, A. Peyman, Chem. Rev., 90, 1990, 544 - 584). Verschiedene Substanzen befinden sich in diesem Zusammenhang als potentielle Therapeutika von Viruskrankheiten, Krebs etc. in diversen klinischen Phasen.

Ein gutes Antisense Molekül sollte dabei u.a. den folgenden Anforderungen genügen:
1. Es sollte ohne Zuhilfenahme sog. Transfektionsreagentien oder Liposomen eine gute Zell- und Zellkerngängigkeit aufweisen;
2. Es sollte zur Erreichung einer ausreichenden Bioverfügbarkeit Nuclease- und Peptidase-Resistenz aufweisen; und
3. Es sollte die Sequenz des natürlichen Sense Stranges präzise erkennen.

Als ein vielversprechendes Antisense und Antigen Polymer hat sich die von Nielsen et al. beschriebene PNA erwiesen (Science 1991, 254, 1497 - 1500; WO 92/20702), die auch als molekularbiologisches Werkzeug vielfältige Anwendung gefunden hat. Dies ist vor allem auf die hohe Affinität der PNA zum Sense Strang (DNA, RNA) bei sehr guter Sequenzspezifität zurückzuführen. Die PNA kann wesentlich besser Mismatches in Sequenzen identifizieren als natürliche DNA oder RNA. Weiterhin vermögen Pyrimidin-reiche PNA-Stränge die DNA-Doppelhelix aufzuwinden und eine (PNA)₂DNA Tripelhelix zu bilden. Solche Strukturen sind potentielle Translations- und Replikationskomplexmimetika und man könnte damit in der Lage sein, gezielt Gene ein- und auszuschalten. Für eine in vivo Anwendung müssen aber noch diverse Eigenschaften der PNA verbessert und optimiert werden. Z.B. ist die PNA nicht zellgängig. Die erwähnte (PNA)₂DNA Tripelhelix Bildung findet nur bei Pyrimidin-reichen PNA-Strängen und nur unter unphysiologischen Salzkonzentrationen statt. PNA's aggregieren und sind schlecht wasserlöslich. PNA - selbst ein polares Polymer - bindet sowohl parallel als auch antiparallel mit ähnlichen Bindungskonstanten an die Zielstruktur DNA oder RNA. Es wird auch über starke cytotoxische Effekte der PNA berichtet (EP 0 672 677 A2).

Wie sich gezeigt hat, werden neue verbesserte PNA's am effektivsten durch Screening einer großen Zahl von Varianten gefunden (S. Jordan et al., Bioorganic & Medicinal Chemistry Letters, 1997, 7, 681 und 687). Zur systematischen und schnellen Herstellung vieler Analoga erscheinen die bisherigen beschriebenen Methoden der PNA Synthese, die auf sequentiellen Zweikomponentenreaktionen von Monomeren beruhen, welche ihrerseits über viele Schritte aus geeigneten käuflichen Vorstufen synthetisiert werden müssen, nicht optimal geeignet (M. Egholm et al., Journal of the American Chemical Society, (1992) 114, 1895).

Es ist daher die Aufgabe der vorliegenden Erfindung, ein Verfahren bereitzustellen, mit dem komplexe Mono-, Oligo- und Polymere, die Nucleobasen als Seitengruppen aufweisen, und insbesondere PNA's mit einer großen Variationsfähigkeit hergestellt werden können.

Es ist eine weitere Aufgabe der vorliegenden Erfindung, eine Komponente bereitzustellen, mit der die Diversität der MCRs und insbesondere von Verfahren zur Herstellung von Nucleobasen als Seitengruppen aufweisenden Polymeren erheblich erhöht werden kann und komplexe Moleküle aufgebaut werden können, d.h. eine Komponente, die in einer MCR-artigen Reaktion umgesetzt werden kann. Derartige Komponenten müssen die folgenden Voraussetzungen erfüllen:
1. Sie müssen leicht und billig herstellbar sein;
2. Die in ihnen verwendeten Schutzgruppen müssen leicht, d.h. unter milden Bedingungen abspaltbar sein;
3. Die in ihnen verwendeten Schutzgruppen müssen unter üblichen Reaktionsbedingungen zur Umsetzung anderer funktioneller Gruppen stabil sein;
4. Die Komponenten müssen so aufgebaut sein, daß nach der Entfernung einer Schutzgruppe funktionelle Gruppen freigesetzt werden, die für eine MCR geeignet sind, ohne daß andere Gruppen z.B. auch funktionelle Gruppen der Komponenten oder der Reaktionspartner modifiziert werden.

Erfindungsgemäß wird ein Verfahren zur Herstellung von Verbindungen der Formel (I) offenbart 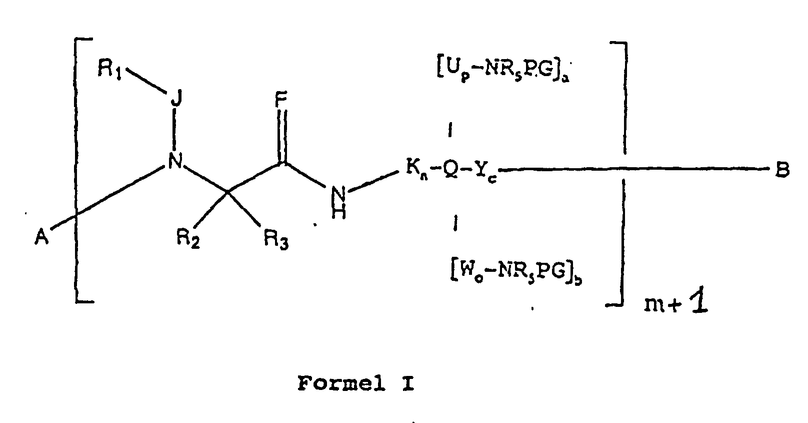 dadurch gekennzeichnet, daß Verbindungen der Formeln

A- NH₂ II

R₁ -G (III) III

R₂-C(O)-R₃ IV

und 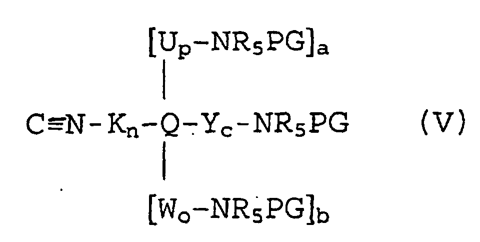 in einem ersten Schritt gegebenenfalls gleichzeitig miteinander umgesetzt werden,
gegebenenfalls eine oder mehrere Schutzgruppen entfernt werden
und die Reaktion m-mal wiederholt wird,
wobei das Produkt des jeweils vorangegangenen Schrittes nach dem ersten Schritt anstelle der Verbindung mit der Formel II eingesetzt wird,
wobei
m 0 oder eine ganze Zahl von 1 bis 1000, vorzugsweise 1 bis 300, stärker bevorzugt 1 - 100, insbesondere 1 - 50, 1 - 30, 1 - 10 oder 1 - 5 ist,
A ein in der Ugi Reaktion üblicher Rest der Aminkomponente wie ein Wasserstoffatom, ein Substituent, (Cyclo-)Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein Saccharid oder Oligosaccharid, ein Antisensepolymer, ein Peptid, ein Antikörper-Konjugat, ein synthetisches Polymer oder eine durch Linker für die Polymersynthese modifizierte Oberfläche ist,
B ein Wasserstoffatom, ein Substituent, (Cyclo-)Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein die Zellaufnahme erleichterndes Agens, ein Saccharid oder Oligosaccharid, ein Antisensepolymer, ein Peptid, ein Antikörper-Konjugat, ein synthetisches Polymer oder eine durch Linker für die Polymersynthese modifizierte Oberfläche oder ein Rest

[Uₚ-NR₅PG]ₐ

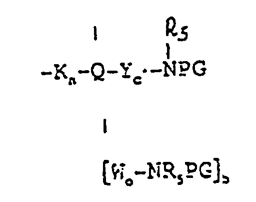

der Verbindung V ist,
R1-G aus Strukturen der folgenden Formeln ausgewählt wird: 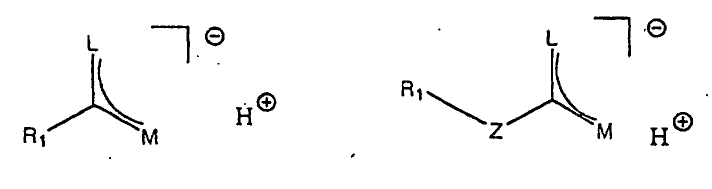 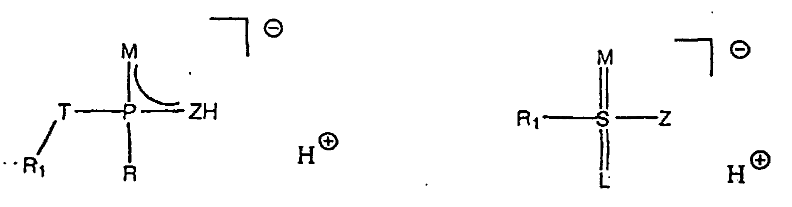 wobei die Gruppe R₁-G über einen molekularen Spacer über R₁ oder R oder R₄ mit der Verbindung der Formel IV verknüpft sein kann;
R₁ und R unabhängig voneinander ein in der Ugi Reaktion üblicher Rest der Säurekomponente wie H, ein Substituent, (Cyclo-) Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein Saccharid oder Oligosaccharid, ein Antisenspolymer, ein Peptid, ein Antikörper-Konjugat, ein synthetisches Polymer oder eine durch Linker für die Polymersynthese modifizierte Oberfläche, oder von den natürlichen Nucleobasen oder von synthetischen Nucleobasen abgeleitete Reste sind;
L, M, T und Z unabhängig voneinander O, S oder NR₄ bedeuten, wobei R₄ H, Fluor, (Cyclo-) Alkyl, (Cyclo-)Alkenyl, (Cyclo-)Alkinyl, Aroyl, Heteroaroyl, Heterocyclus oder -O(Cyclo-)Alkyl, - OAroyl, -S(Cyclo-)Alkyl, -SAroyl bedeuten;
R₂ und R₃ unabhängig voneinander ein in der Ugi Reaktion üblicher Rest der Oxokomponente wie H, ein Substituent, (Cyclo-)Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein über einen Aminspacer verknüpftes Saccharid oder Oligosaccharid, ein Antisensemolekül, ein Peptid, ein Antikörper-Konjugat, ein synthetisches Polymer oder eine durch Linker für die Polymersynthese modifizierte Oberfläche ist,
PG unabhängig voneinander gegebenenfalls orthogonale, an sich übliche, leicht abspaltbare (temporäre), Schutzgruppen für Amingruppen aus der Klasse der, der N-Silylderivate, oder darstellen; oder aus der Gruppe ausgewählt sind:
tert Boc-, Alloc-, Fmoc-, Moz-, z-, Tr-, MMTr-, DMTr-, Pixyl-, und TBDMS- Schutzgruppe,
die Reste R₅ unabhängig voneinander Wasserstoffatome, unsubstituierte oder substituierte Alkyl-, Cycloalkyl-, Alkoxyalkyl- oder Arylgruppen oder Heterocyclen darstellen;
die Reste U, W, K und Y unabhängig voneinander unsubstituierte oder substituierte Alkyl-, Alkenyl-, Alkinyl-, Alkanoyl-, Alkoxyalkanoyl-, Cycloalkyl- oder Arylgruppen, unsubstituierte oder substituierte Heterocyclen oder die Gruppe NR₅ darstellen,
wobei R₅ wie vorstehend definiert ist;
a, b, c, n, o und p unabhängig voneinander eine ganze Zahl von 0-10 vorzugsweise 0-5 sind;
Q eine unsubstituierte oder substituierte Alkyl-, Aryl-, Alkenyl-, Alkinyl-, oder Cycloalkyl-Gruppe, oder einen unsubstituierten oder substitierten Heterocyclus, oder eine Gruppe der Formel SO₂ darstellt,
F eine Oxo, Thio, Seleno oder Iminogruppe darstellt, und
J aus den folgenden Strukturen ausgewählt wird, wobei der obere vertikale Strich die Bindung zu R₁ und der untere vertikale Strich die Bindung zum Stickstoffatom der Hauptkette des Polymers in der allgemeinen Formel I darstellt: 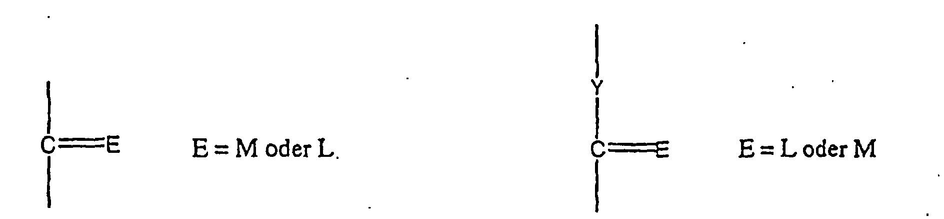 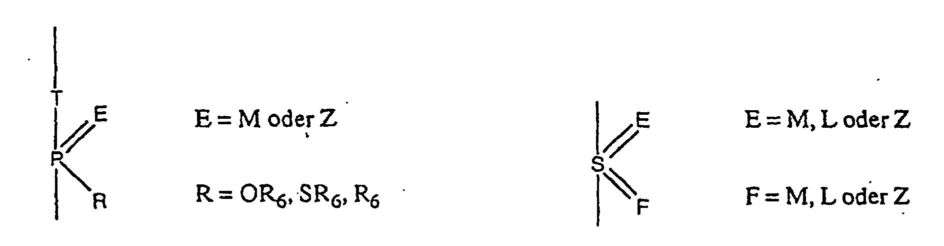 wobei R₆ H, einen Substituent, (Cyclo-)Alkyl, (Cyclo-)Alkenyl, (Cyclo-)Alkinyl, Aroyl, Heteroaroyl, Heterocyclus darstellt, mit der Maßgabe, daß mindestens einer der Reste R oder R₁ in der Verbindung der Formel I ein von einer natürlichen oder synthetischen Nucleobase abgeleiteter Rest ist (Anspruch 1).

In einer bevorzugten Ausführungsform ist das Verfahren dadurch gekennzeichnet, daß: die Fluoreszenzmarker Fluorescein, Texas Rot, Lissamin Rhodamin, Cyanin oder Rhodamin, die Intercalatoren Psoralen, Acridin, Phenanthrolin, ein Phenanthrolin-Metall Komplex oder Ellipticin sind, die Antibiotika Endiine, β-Lactame, Tetracycline, Anthracycline, Polyether, Mitomycin-artige, Phosphomycin-artige, Macrolide, Bleomycin-artige oder ein Aminoglycosid sind, der Minor Groove Binder Netropsin oder Distamycin ist, das Polyamin ein Spermidin-artiges Polyamin ist, das Antisensepolymer ein DNA- (5' oder 3' verknüpft) oder RNA-Strang (5' oder 3' verknüpft) oder ein Phosphothioat ist, das Peptid N- oder C-terminal verknüpft ist, das Antikörper-Konjugat aus Antikörperkonjugaten ausgewählt wird, die zellspezifische Aufnahme gewähren, spezifische Carriersysteme ansprechen oder Endocytose bewirken, das synthetische Polymer CPG, Wang, oder Tentagel ist, die natürlichen Nucleobasen Adenin, Thymin, Guanin, Cytosin oder Uracil sind und die synthetischen Nucleobasen Pseudouracil, 5-Propinyluracil, 5-Hexenyluracil, 5-Fluorcytidin, 5-Hydroxymethyluracil, 5-Methylcytidin, 5-Bromcytidin und Verbindungen der folgenden Formeln sind 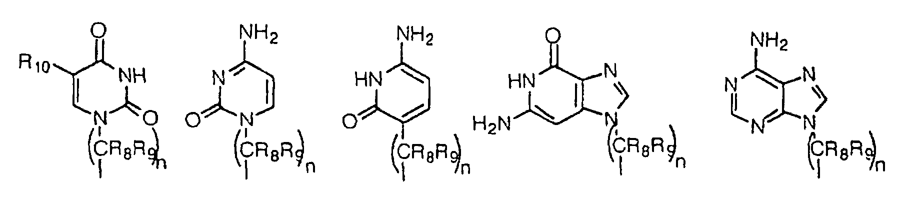 wobei R₈ und R₉ unabhängig voneinander H, (Cyclo-)Alkyl, (Cyclo-)Alkenyl, (Cyclo-)Alkinyl, Aroyl, Heteroaroyl, Heterocyclus, Chlor oder Fluor sind,
R₁₀ = Fluor, Brom, Jod, Chlor, Alkinyl, (Cyclo-)Alkyl, Aroyl, Heteroaroyl oder H ist, und n = 1 - 20, bevorzugt 1 - 10 und besonders bevorzugt 1 - 5 ist, wobei
die Seitengruppen der Basen mit dem Fachmann bekannten Schutzgruppen, wie z.B.tert-Butylbenzoyl, p-Methoxybenzyl, iso-Butanoyl geschützt sein können.

Alle bisherigen und im folgenden genannten funktionellen Einheiten wie Antibiotika, Groove Binder, Antisensemoleküle, Steroide, Antikörperkonjugate, Interkalatoren und Oligosaccharide können über einen geeignet konstruierten Spacer an das Hauptpolymer gebunden werden. Geeignet meint mindestens eine funktionelle Gruppe der Ugi-Reaktion wie Oxo-, Säure-, Amin- oder Isocyano-funktion enthaltend. Geeignet kann aber auch eine sonstige funktionelle Gruppe bedeuten, über die der Fachmann zwei molekulare Einheiten miteinander verbindet.

Die Schutzgruppen PG sind an sich übliche leicht abspaltbare (temporäre) Schutzgruppen für Amingruppen. Ansonsten müssten zur ihrer Abspaltung zu drastische Bedingungen eingesetzt werden, welche zu unerwünschten Nebenreaktionen oder sogar zur Zersetzung der Reaktionsprodukte führen können. Bei den Schutzgruppen PG, handelt es sich um N-Silylschutzgruppen oder Schutzgruppen, wie tert. Boc-, Alloc-, Fmoc-, Moz-, Z-, Tr-, MMTr-, DMTr-, Pixyl-, TBDMS-Schutzgruppen.

Die Reste R₅ stellen bevorzugt unabhängig voneinander Wasserstoffatome, oder unsubstituierte oder substituierte Alkyl-, Aryl-, Cycloalkyl-, Alkoxyalkylgruppen oder Heterocyclen, insbesondere sterisch wenig anspruchsvolle Gruppen, wie Alkyl, Aryl, Cycloalkyl, Heterocyclen und besonders bevorzugt Wasserstoffatome dar, primäre Amine meist reaktiver sind als sekundäre Amine [I. Ugi, Angew. Chem. **74,** 9 (1962)].

Die Reste U, W, K und Y stellen bevorzugt unabhängig voneinander unsubstituierte oder substituierte Alkyl-, Cycloalkyl- oder Arylgruppen und besonders bevorzugt Methylen-, Ethylen-, Propylen-, Heptylen-, Octylen-, Nonylen-, Oxymethylen-, Oxyethylen-, Cyclohexyl-, Phenylgruppen und Heterocyclen dar.

Die Indices a, b, m, n, o und p sind bevorzugt unabhängig voneinander ganze Zahlen von 0-50, stärker bevorzugt von 0-20 bzw. von 0-10, noch stärker bevorzugt von 0-5 und besonders bevorzugt von 0-3 und am stärksten bevorzugt sind a, b, p und o 0 und sind m und n 0, 1 oder 2.

Der Rest Q stellt bevorzugt mindestens eine unsubstituierte Alkyl-, Aroyl- oder Arylgruppe oder einen unsubstituierten oder substituierten Heterocyclus und besonders bevorzugt Methylen-, Ethylen-, Propylen-, Oxymethylen-, Oxyethylen-, Dioxymethylen-, 1,2-Dioxyethylen-, Cyclohexyl-, Phenylgruppen, N und Heterocyclen dar.

Erfindungsgemäß werden also auch Verbindungen der Formel V bereitgestellt. Bevorzugte Verbindungen der Formel V weisen die folgende Struktur auf:

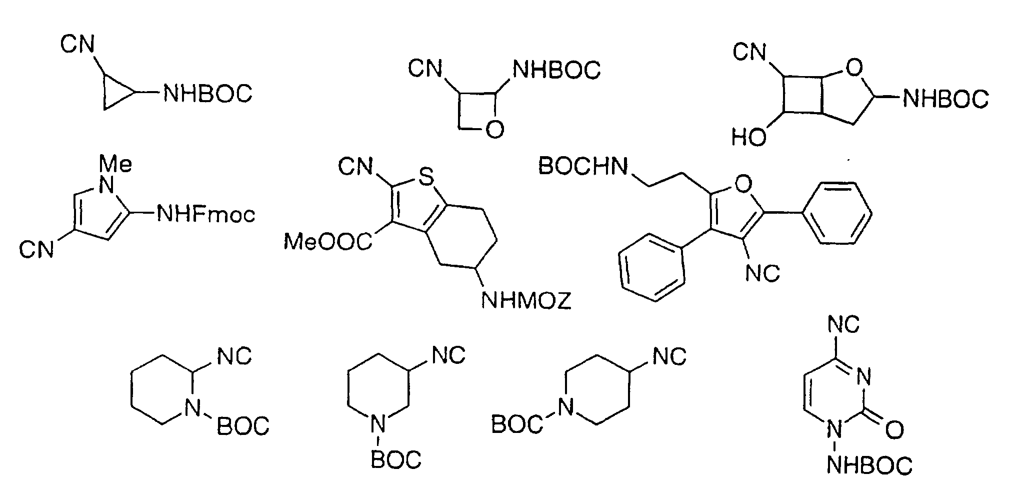

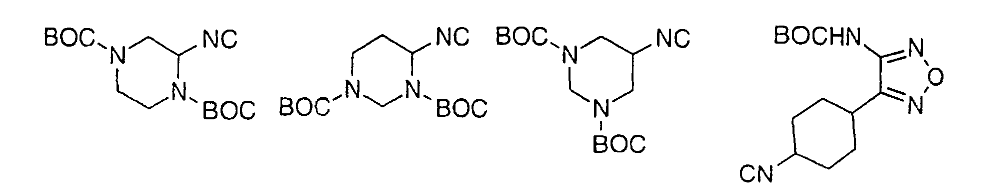

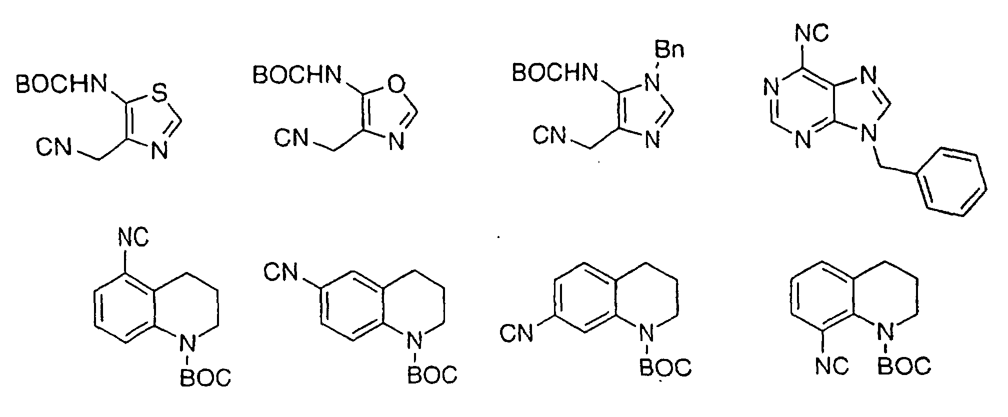

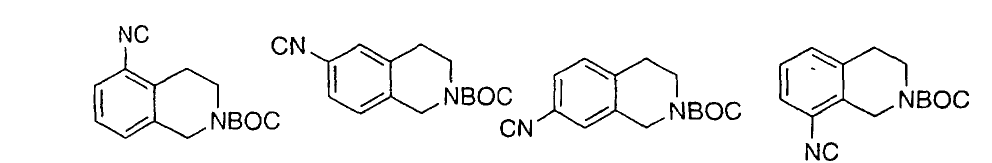

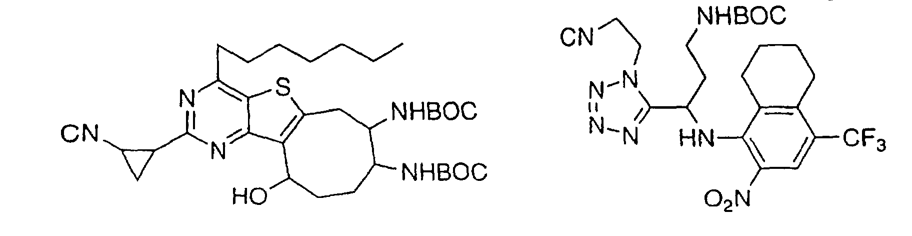

Verbindungen der Formel V können erfindungsgemäß dadurch hergestellt werden, daß eine Verbindung der Formel VII 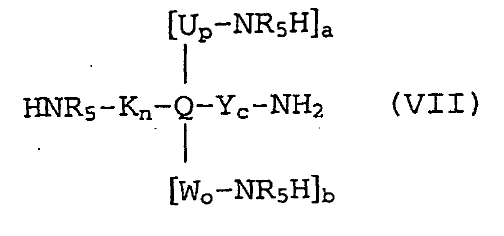 mit den wie vorstehend definierten Gruppen PG, die voneinander unabhängig sein können, mit üblichen Verfahren geschützt wird, wodurch eine Verbindung der Formel VIII hergestellt wird 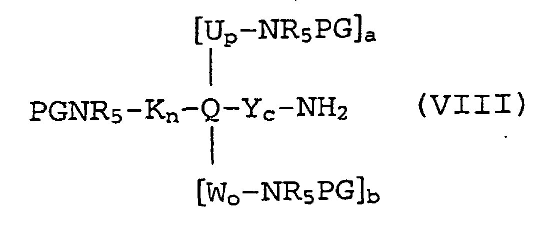 wobei die Reste und Indices wie vorstehend definiert sind, und dann die Verbindung der Formel VIII mit üblichen Verfahren zu einem Isonitril der Formel V umgesetzt wird. Derartige übliche Verfahren zur Umsetzung von Verbindungen der Formel VIII zu Isonitrilen der Formel V sind z.B. in W.P. Weber, G.W. Gokel, Tetrahedron Lett. 1972, 1637; W.P. Weber, G.W. Gokel, I.K. Ugi, *Angew. Chem*. **84,** 587 (1972); *Angew. Chem. Int. Ed. Engl*. **11,** 530 (1972) beschrieben. Verbindungen der Formel VII sind im Handel erhältlich.

In einer weiteren Ausführungsform wird die Verbindung der Formel VIII mit üblichen Formylierungsreagenzien zu einer Verbindung der Formel IX umgesetzt: 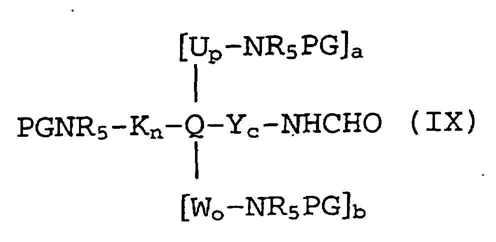 die dann unter üblichen Bedingungen zu einer Verbindung der Formel V umgesetzt wird, wobei die Reste und Indices wie vorstehend definiert sind. Die Formylierung mit derartigen üblichen Formylierungsreagentien ist z.B. in U. Schöllkopf et al., *Liebigs Ann.* **89,** 351-360 (1977) beschrieben. Ein übliches Verfahren zur Umsetzung einer Verbindung der Formel IX zu einer Verbindung der Formel V ist eine Wasserabspaltung, wie z.B. von I. Hagedorn, H. Tönjes, *Pharmazie* **11**, 409 (1956); I. Ugi, R. Meyr, *Angew. Chem.* **70,** 702 (1958); H.M. Walborsky, G.E. Niznik, *J. Org. Chem*. **37,** 187 (1972); I. Ugi, W. Betz, U. Fetzer, K. Offermann, *Chem. Ber*. **94**, 2814 (1961) I. Ugi, R. Obrecht, R. Herrmann, *Synthesis* 1985, 400-402; G. Gokel, D. Marquarding, P. Hoffmann, I. Ugi in Isonitrile Chemistry; I. Ugi (Ed.), Academic Press, New York, London 1971, 9 beschrieben. Bevorzugt werden dabei Phosphoroxychlorid und eine geeignete Base wie Triethylamin oder Diisopropylamin eingesetzt.

In einer weiteren Ausführungsform wird ausgehend von Verbindungen der Formel X 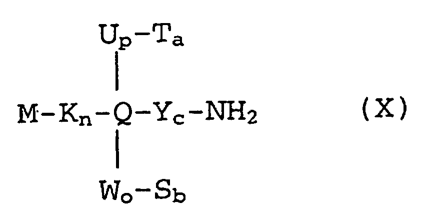 wobei die Reste und Indices wie vorstehend definiert sind, zunächst die Aminfunktion z.B. nach einer o.g. Methode formyliert, anschließend eine oder mehrere der Funktionalitäten M, T und S, die unabhängig voneinander Halogene oder Hydroxyfunktionen, bevorzugt Chlor, Brom und Hydroxyl bedeuten, nach einem üblichen Verfahren in eine oder mehrere Azidfunktionen umgewandelt, diese Azide nach einem üblichen Verfahren in die entsprechenden Amine konvertiert und diese dann mit üblichen Verfahren mit o.g. Schutzgruppen versehen, um zu Verbindungen der Formel IX zu gelangen. Ebenso können die Verbindungen der Formel X nach Formylierung.und Umwandlung der Gruppen M, T und S in die entsprechenden Azide z.B. nach o.g. Methoden zuerst in die entsprechenden Isonitrile umgesetzt werden, die Azidfunktionen nach einem üblichen Verfahren in Amine konvertiert und dann mit o.g. Schutzgruppen versehen werden, um zu Verbindungen der Formel V zu gelangen.

Erfindungsgemäß wird weiter die Verwendung von Verbindungen der Formel V offenbart:

Bei ihrer Verwendung können die erfindungsgemäßen Verbindungen zunächst z.B. im Rahmen einer üblichen MCR wie z.B. vom Ugi- oder Passerini-Typ mit 2, 3 oder mehr weiteren Verbindungen wie Aldehyden, Aminen und Carbonsäuren umgesetzt werden. Danach kann durch Abspaltung von mindestens einer Schutzgruppe mindestens eine funktionelle Gruppe wie ein primäres oder sekundäres Amin oder eine Hydrazingruppe freigesetzt werden, die dann z.B. im Rahmen einer weiteren wie vorstehend beschriebenen MCR oder einer klassischen Zweikomponentenreaktion weiter umgesetzt werden kann. Dadurch kann auch durch wiederholten Einsatz der erfindungsgemäßen Verbindungen eine Vielzahl hochkomplexer Moleküle hergestellt werden. Durch die selektive Abspaltung von Schutzgruppen und genau definierte Reaktionspartner ist es weiterhin möglich, eine Vielzahl ansonsten nicht oder nur schwer zugänglicher Moleküle zu synthetisieren. Weiter können die erfindungsgemäßen Verbindungen zur Herstellung von peptidischen Nukleinsäurepolymeren (PNA) verwendet werden.

In der Beschreibung und den Ansprüchen werden die folgemden Definitionen zu Grunde gelegt:
Alkyl oder "Alk" oder "Alkan" auch als Wortbestandteile: Kettenlänge C1 bis C100, bevorzugt C1 bis C20, stärker bevorzugt C1 bis C10, noch stärker bevorzugt C1 bis C6 und am stärksten bevorzugt C1 bis C4, linear oder verzweigt, substituiert (wie unten definiert) oder unsubstituiert, Cycloalkyl oder Cycloalkan: Ringgröße C3 bis C20, bevorzugt C3 bis C9, stärker bevorzugt C3 bis C7, am stärksten bevorzugt C5, C6 oder C7, substituiert (wie unten definiert) oder unsubstituiert,
Alkenyl: 1 bis 5, vorzugsweise 1 oder 2 konjugierte oder nicht konjugierte Doppelbindungen enthaltendes Alkyl (mit mindestens 2 C-Atomen) oder Cycloalkyl,
Alkinyl: 1 bis 5, vorzugsweise 1 oder 2 konjugierte oder nicht konjugierte Dreifachbindungen enthaltendes Alkyl (mit mindestens 2 C-Atomen) oder Cycloalkyl,
Heterocyclus: 3 bis 7 gliedrige, vorzugsweise 5 oder 6 gliedrige, Heterocyclen mit 1, 2, 3 oder gegebenenfalls 4 Heteroatomen, wie N, O oder S wie Z. B. substituiertes (wie unten definiert) oder unsubstituiertes Oxiran, Thiiran, Aziridin, Oxaziridin, Oxetan, Thietan, Azetidin, Tetrahydrofuran, Dihydrofuran, Tetrahydrothiophen, Dihydrothiophen, Pyrrolidin, Dihydropyrrol, 1,3-Dioxolan, 1,3-Dithiolan, Imidazolidin, Oxazolidin, Thiazolidin, 2H-Pyran, 4H-Pyran, Tetrahydropyran, 2H-Thiopyran, 4H-Thiopyran, Tetrahydrothiopyran, Piperidin, Morpholin, 1,4-Dioxin, 1,4-Dioxan, 1,4-Dithiin, 1,4-Dithian, Piperazin, Oxepan, Thiepan, Thiepin, 1H-Azepin, 2H-Azepin, Azepan,
Aroyl: substituiertes (wie unten definiert) oder unsubstituiertes Benzol, Naphthalin, Anthrazen, Biphenyl, Triphenyl, Azulen, Ferrocen, Cyclopropenylium,
Heteroaroyl: 5-6 gliedrige heterocyclische aromatische Heterocyclen mit 1, 2 oder 3 Heteroatomen wie z.B. substituiertes (wie unten definiert) Pyrrol, Furan, Thiophen, Pyrazol, Isoxazol, Isothiazol, Imidazol, Oxazol, Thiazol, 1,2,4-Triazol, 1,2,4-Oxadiazol, 1,2,4-Thiadiazol, 1,2,5-Oxadiazol, 1,2,5-Thiadiazol, Tetrazol, Pyridin, Pyrylium, Thiapyrylium, Pyridazin, Pyrimidin, Pyrazin, 1,2,3-Triazin, 1,2,4-Triazin, 1,3,5-Triazin, 1,2,3,4-Tetrazin, 1,2,3,5-Tetrazin, 1,2,4,5-Tetrazin, Indol, Cumaron, Thionaphthen, Carbazol, Bibenzofuran, Dibenzothiophen, 1*H*-Indazol, Indoxazol, Benzo[d]isothiazol, Anthranil, Benzimidazol, Benzoxazol, Benzothiazol, Benzotriazol, Chinolin, Isochinolin, Benzopyrylium, Thiabenzopyrylium, Acridin, Benzo[g]chinolin, Benzo[g]isochinolin, Benzo[c]chinolin, Cinnolin, Phthalazin, Chinazolin, Chinoxalin, Phenazin, Benzo[g]cinnolin, Benzo[g]chinazolin, Benzo[g]chinoxalin, 1,5-Naphthyridin, 1,6-Naphthyridin, 1,7-Naphthyridin, 1,8-Naphthyridin, 2,6-Naphthyridin, 2,7-Naphthyridin, 1,7-Phenanthrolin, 1,8-Phenanthrolin, 1,9-Phenanthrolin,1,10-Phenanthrolin, Indolizin, 4H-Chinolizin, Carbolin, Ergolin, Purin, Pteridin, Alloxazin, Flavin,
Substituent oder "substituiert mit": -H, -OH, -Rₐ, -O- (Cyclo-)Alkyl, -O-Aryl, -O-Heteroaroyl, -O-Heterocyclus, -NH₂, -NO₂, -CN, -N₃, -CNRₐNR_{b}R_{c}, -NRₐR_{b}, NRₐR_{b}R_{c}⁺, Fluor, Chlor, Brom, α-, β-, bis ω-Aminosäureester, -NRₐCOR_{b}, -NRₐCOXR_{b} (X = -O, -NR, -PO_{0,2,3,4}R, -SO_{0,1,2,4,}R, -NRₐNR_{b}R_{c}), -CORₐ, -COORₐ, -OCOORₐ, -CONRₐR_{b}, -OCONRₐR_{b}, -NR_{c}CONRₐR_{b}, -Rₐ-O-R_{b}, -R_{c}-NRₐR_{b}, -Rₐ-S-R_{b}, -Rₐ-SO-R_{b}, -Rₐ-S(O)₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, -NRₐCOR_{b}-O-R_{c}, -ORₐ-S-R_{b}, -NRₐR_{b}-S-R_{c}, -SO_{1,2,3,4}Rₐ-S-R_{b}, -CORₐ-S-R_{b}, -OCORₐ-S-R_{b}, -OCORₐ-S-R_{b}, NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b}, -NR_{c}POORₐOR_{b},
wobei Rₐ, R_{b}, R_{c} und R_{d} unabhängig voneinander wie oben definiert (Cyclo-)Alkyl, Alkenyl, Akinyl, Aroyl, Heteroaroyl, ein Heterocyclus, Aralkyl, Aralkenyl oder Perhalogenalkyl sein können und wobei Rₐ, R_{b}, R_{c} und R_{d} substituiert sein können.

Das erfindungsgemäße Verfahren betrifft also die Herstellung von Mono-, Oligo- und Polymeren, die Nucleobasen als Seitengruppen aufweisen, insbesondere von Peptide Nucleic Acid (PNA) und deren Varianten mittels Multi Komponenten Reaktionen (MCR's), speziell MCR's auf Isocyanid-Basis wie der Ugi-Reaktion. Dabei wird nicht wie bei herkömmlichen Verfahren auf Monomere zurückgegriffen sondern mit kleineren Bausteine als bisher üblich gearbeitet, sog. Submonomeren. Das hier vorgestellte Verfahren erlaubt eine verbesserte Synthese der bisher beschriebenen PNA Varianten und v.a. die rasche Synthese neuer bisher nicht beschriebener Varianten mit potentiell verbesserten und/oder neuen Eigenschaften. Aufgrund der Multikomponenten-Natur dieses Verfahrens ist der gleichzeitige Einbau vieler verschieden gearteter Reste in quasi kombinatorischer Manier z.B. in das Polyamid-Rückgrat der PNA, sowie mit geeigneten weiter unten beschriebenen Synthesebausteinen der Aufbau von (PNA)₂-, (PNA)(DNA)-, (PNA)(RNA)-, (PNA)(Peptid)-, (PNA)₂(DNA)-, (PNA)((Oligo)Saccharid)-, (PNA)₂(DNA)(Peptid)-Chimaeren etc., sowie der Aufbau von veränderten PNA-Polymerrückgraten wie z.B. Thioamiden möglich. Nach dem beschriebenen Verfahren hergestellte kürzere Oligomere oder gar Monomere sind potentiell pharmakologisch oder agrochemisch relevante Wirkstoffe.

Das hier beschriebene Verfahren beruht auf Isocyanid-gestützten Multikomponenten Reaktionen (MCR's) vom Typ der Vier Komponenten Kondensation oder Ugi-Reaktion (Isocyanide Chemistry, (I. Ugi, ed.), Wiley, New York, 1971; I. Ugi, R. Karl, in: Comprehensive Organic Synthesis), (B.M.Trost, C. H. Heathcock (ed.), Vol. II, 1083 - 1109, Pergamon Press, New York 1991). In dieser Reaktion reagieren die vier Eduktkomponenten Isocyanid, Oxoverbindung (Aldehyde oder Ketone), aminartige Verbindungen (z.B. Ammoniak, primäre Amine, sekundäre Amine, Hydrazin und Derivate, Hydroxylamin und Derivate) und geeignete Säurekomponenten (z.B. Carbonsäuren, Kohlensäuremonoester, Wasser, Thiosulfat, Selenwasserstoff, Stickstoffwasserstoffsäure, Cyansäure, Thiocyansäure) zu einheitlichen Produkten, deren zentrales Grundgerüst wesentlich von der Natur der Säurekomponente abhängt. Bemerkenswerterweise können die Reste der einzelnen Komponenten ohne Reaktivitätsverlust in weiten Grenzen variiert werden. So reagieren sterisch anspruchsvolle oder kleine, aromatische, heteroaromatische wie auch aliphatische oder heterocyclische, elektronenziehende oder elektronenschiebende Edukte gleichermaßen in der Ugi Reaktion. Verwandte auf Isocyaniden basierende MCR's sind die Passerini Reaktion (I. Ugi in, Isocyanide Chemistry, (I. Ugi, ed.), Wiley, New York, 1971), sowie eine Reihe von Heterocyclensynthesen (S. Marcaccini, T. Torroba, OPPI, 143.).

Erfindungsgemäß einsetzbare Verbindungen weisen z.B. die folgenden Strukturen auf:
Imine: 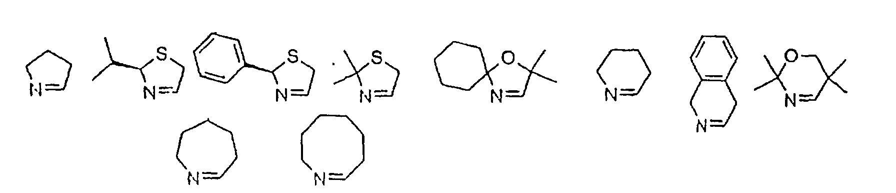 Isocyanide: 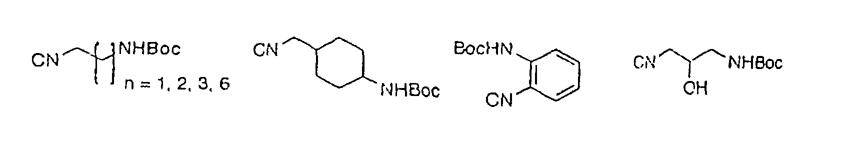 Carbonsäuren:

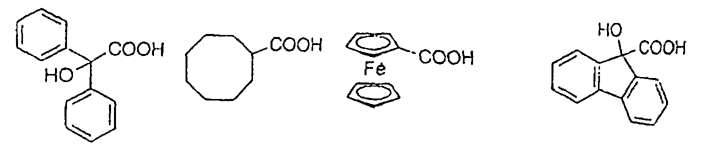

Diese Verbindungen können z.B. in der folgenden Umsetzung eingesetzt werden:

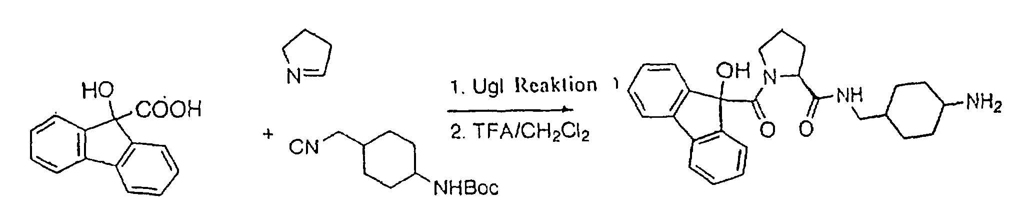

Mit Hilfe des erfindungsgemäßen Verfahrens können auch Homound Heteropolymere der allgemeinen Formel I wie in Schema I gezeigt hergestellt werden.

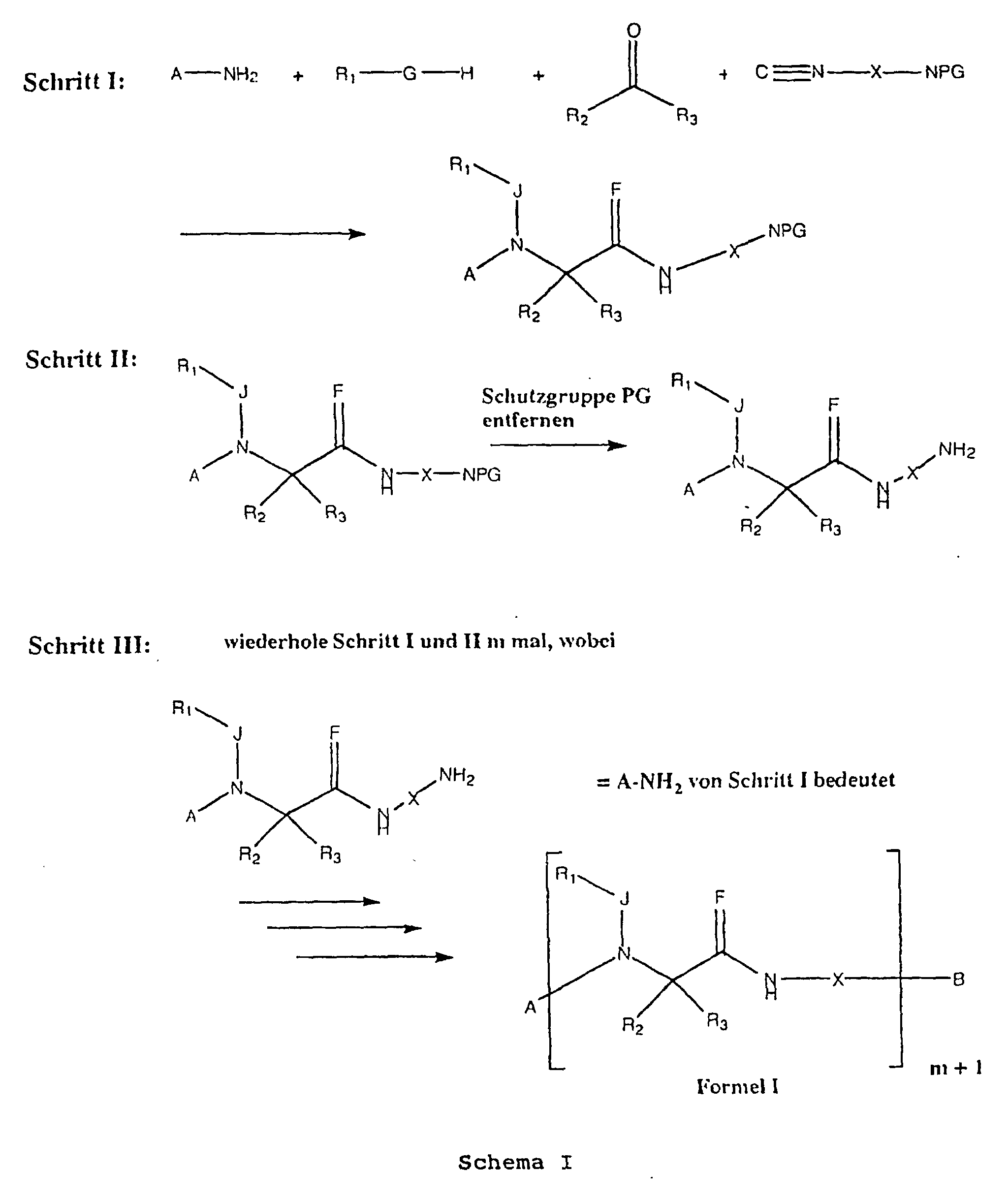

Das Verfahren zur Herstellung von Homo- und Heteropolymeren der allgemeinen Formel I ist dadurch gekennzeichnet, daß vier verschiedene Verbindungen mit geeigneten funktionellen Gruppen gegebenenfalls synchron gegebenenfalls mehrfach miteinander umgesetzt werden. Nach dem ersten Schritt, d.h. der gegebenenfalls synchron erfolgenden Umsetzung der Verbindungen II, III, IV und V, wird das Produkt des jeweils vorangegangenen Schrittes dann an Stelle der Verbindung der Formel II eingesetzt. Um zu Heteropolymeren zu gelangen, weisen die Verbindungen nicht in allen Syntheseschritten die gleichen Reste oder funktionellen Gruppen auf.

Die Synthesen der unter Formel I gezeigten Verbindungsklassen kann auf Oberflächen, in flüssiger Phase oder an polymeren Trägern durchgeführt werden.

Die Synthese der Verbindungen der Formel I kann weiter auf sogenannten Chips erfolgen:
dabei wird ein Substrat hergestellt, auf dem in einem ersten Bereich eine Verbindung, ausgewählt aus Verbindungen der Formel II, III, IV und V, aufgebracht wird, die Schutzgruppen aufweisen, welche mit Aktivatoren entfernt werden können;
dieser Schritt gegebenenfalls auf anderen Bereichen der Oberfläche jeweils mit einer anderen der (eine) entsprechende Schutzgruppe(n) aufweisenden Verbindungen II, III, IV und V wiederholt,
ein Bereich des gegebenenfalls eine oder mehrere der Verbindungen II, III, IV und V aufweisenden Oberfläche einem Aktivator ausgesetzt, um mindestens eine Schutzgruppe zu entfernen,
dieser Bereich einer Verbindung der Formel II, III, IV oder V ausgesetzt, die wiederum eine lichtempfindliche Schutzgruppe aufweist,
und dieser Schritt gegebenenfalls in anderen Bereichen mit einer beliebigen Kombination der Verbindungen der Formel II, III, IV und V wiederholen.

Das Substrat kann dabei ein polymerisierter Langmuir Blodgettfilm, ein funktionalisiertes Glas, Germanium, Silizium, Polymere, (Poly-)Tetrafluorethylen, Polystyrol, Galliumarsenit oder Kombinationen davon sein;
als Aktivatoren kommen Ionenstrahlen, Elektronenstrahlen, γ-Strahlen, Röntgenstrahlen, Ultraviolette Strahlen, Licht, Infrarotstrahlen, Mikrowellen, elektrische Ströme, Radiowellen und Kombinationen davon in Betracht.

Als lichtempfindliche Schutzgruppen kommen z.B. orto-Nitrobenzylderivate, 6-Nitroveratryloxycarbononyl, 2-Nitrobenzyloxycarbonyl, Zinnamoylderivate und Gemische davon in Betracht.

Weiter wird bzgl. der zu verwendenden Substrate der einzusetzenden Aktivatoren, der lichtempfindlichen Schutzgruppen und der Bedingungen, bei denen die einzelnen Schritte durchgeführt werden, auf die folgenden Druckschriften voll inhaltlich Bezug genommen: WO 90/15070, WO 91/07087, WO 92/10092, EP 0 728 520.\

Vorteile von PNA-Chips gegenüber DNA-Chips sind u.a. die wesentlich bessere Erkennung von Mutationen in der zu untersuchenden DNA, sowie die größere Stabilität von PNA-Chips verglichen mit DNA-Chips.

Der Aufbau eines DNA- oder RNA-Stranges in A erfolgt nach den dem Fachmann bekannten Verfahren, wie der Triester-Methode, der H-Phosphonat-Methode oder Phosphoamidit-Methode, bevorzugt nach der Standard-Phosphoamidit Methode nach Caruthers (M. H. Caruthers et al., J. Am. Chem. Soc., 103, 3185 (1981)). Als Linkerbaustein zur Verbindung zur herkömmlichen in Schema I synthetisierten PNA kann folgende Verbindung dienen: 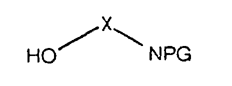 wobei x ein gegebenenfalls substituiertes Alkyl, Cycloalkyl, (Cyclo-)Alkenyl, (Cyclo-)Alkinyl, Aroyl, Heteroaroyl, oder Heterocyclus bedeutet

PG eine Schutzgruppe ist, die mit einem synthetisierten DNA- oder RNA- oder sonstigem Antisense Polymer Strang kompatibel sein muß, und z.B. Dimethoxytrityl, Fmoc, Moc, 5-Dimethoxybenzylcarbamat, o- oder m-Nitrophenylcarbamate wie Nvoc oder 3,4-Dimethoxy-6-nitrobenzylcarbamat oder Phenyl(onitrophenyl)methylcarbamat bedeutet.

Der Aufbau eines Peptidstranges in A erfolgt nach den dem Fachmann bekannten Verfahren, wie der Merrifield Methode (E. Atherton, R. C. Sheppard, Solid Phase Peptide Synthesis - A Practical Approach, IRL Press, New York, 1989). Als Linkerbaustein zur Verbindung zur herkömmlichen in Schema I synthetisierten PNA kann jede natürliche oder synthetische N-geschützte Animosäure dienen. Nach Abspaltung der Schutzgruppe wird im Anschluß an den Peptidstrang ein PNA-Strang nach der im Schema I skizzieren Methodik hergestellt.

Der Aufbau eines Oligosaccharidstranges in A erfolgt nach den dem Fachmann bekannten Verfahren, wie z.B. der Schmidtschen Trichloracetimidat- Methode oder der Ringöffnung von Epoxiden (P. Collins, R. Ferrier, Monosaccharides, Wiley, New York 1996, 415 - 430). Als Linkerbaustein zur Verbindung zur herkömmlichen in Schema I synthetisierten PNA kann z.B. 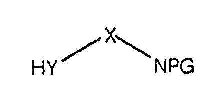 wobei X ein gegebenenfalls substituiertes Alkyl, Cycloalkyl, (Cyclo-)Alkenyl, (Cyclo-)Alkinyl, Aroyl, Heteroaroyl, oder Heterocyclus bedeutet,

Y ein Nucleophil wie S, O, NR mit R = ein gegebenenfalls substituiertes Alkyl, Cycloalkyl, (Cyclo-)Alkenyl, (Cyclo-)Alkinyl, Aroyl, Heteroaroyl, Heterocyclus oder -CHPL bedeutet, wobei P und L unabhängig voneinander -CN, -NC, -COOR, -PO(OR)₂ oder -SO₂R bedeuten,

PG eine Schutzgruppe ist, die mit einem synthetisierten Oligosaccharid Strang kompatibel sein muß, und z.B. Dimethoxytrityl, Fmoc oder Pmoc oder Nvoc bedeuten.

Die Gruppe X in der Formel V kann Ausgangspunkt für eine weitere DNA-, RNA-, PNA- oder Peptid- oder eine andere Oligomer- oder Polymer- Synthese sein.

Der Aufbau eines DNA- oder RNA-Stranges in X erfolgt nach den dem Fachmann bekannten Verfahren, wie der Triester-Methode, der H-Phosphonat-Methode oder Phosphoamidit-Methode, bevorzugt nach der Standard-Phosphoamidit Methode nach Caruthers (M. H. Caruthers et al., J. Am. Chem. Soc., 103 3185 (1981)). Als Linkerbaustein zur Verbindung zur herkömmlichen in Schema I synthetisierten PNA kann z.B. folgende Verbindung der allgemeinen Formel CN-X-NPG dienen: 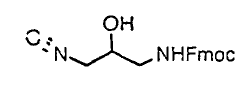 wobei die Hydroxygruppe als Anker für die folgende RNA/DNA oder Phosphothioat Antisense Synthese dient.

Der Aufbau eines Peptidstranges oder eines weiteren PNA Stranges in X erfolgt nach den dem Fachmann bekannten Verfahren, wie der Merrifield Methode (E. Atherton, R. C. Sheppard, Solid Phase Peptide Synthesis - A Practical Approach, IRL Press, New York, 1989) bzw. der in Schema I beschriebenen Methode (PNA). Als Linkerbaustein kann jedes Isocyanid mit einer geschützten Aminfunktion wie z.B. folgendes trifunktionelles Isocyanid der allgemeinen Formel CN-X-NPG dienen:

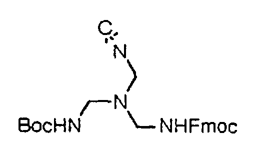

Nach vollzogener Ugi Reaktion mit dem Isocyanid wird z.B. die Fmoc Schutzgruppe abgespalten und ein Peptid- oder PNA-Strang aufgebaut. Nach Beendigung der Seitenketten Synthese wird durch Abspalten der Boc Schutzgruppe die PNA der Hauptkette weiter synthetisiert.

Der Aufbau eines Oligosaccharidstranges in X erfolgt nach den dem Fachmann bekannten Verfahren, wie z.B. der Schmidtschen Trichloracetimidat- Methode oder der Ringöffnung von Epoxiden (P. Collins, R. Ferrier, Monosaccharides, Wiley, New York 1996, 415 - 430). Als Linkerbaustein kann z.B. folgendes trifunktionelles Isocyanid der allgemeinen Formel CN-X-NPG dienen: 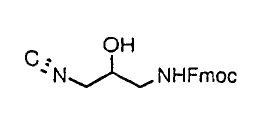 wobei die Glycolysierung an der freien Hydroxyfunktion stattfindet oder folgendes Isocyanid der allgemeinen Formel CN-X-NPG dienen: 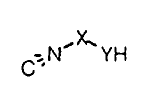 wobei die Glycolysierung über die nucleophile Y-Gruppe stattfindet,
X substituiertes Alkyl, Cycloalkyl, (Cyclo-)Alkenyl, (Cyclo-)Alkinyl, Aroyl, Heteroaroyl, Heterocyclus,
Y ein Nucleophil wie S, O, NR mit R = substituiertes Alkyl, Cycloalkyl, (Cyclo-)Alkenyl, (Cyclo-)Alkinyl, Aroyl, Heteroaroyl, Heterocyclus oder -CHPL ist, wobei P und L unabhängig voneinander -CN, -NC, -COOR, -PO(OR)₂ oder -SO₂R bedeuten.

Der Aufbau eines DNA- oder RNA-Stranges in B erfolgt nach den dem Fachmann bekannten Verfahren, wie der Triester-Methode, der H-Phosphonat-Methode oder Phosphoamidit-Methode, bevorzugt nach der Standard-Phosphoamidit Methode nach Caruthers (M. H. Caruthers et al., J. Am. Chem. Soc., 103 3185 (1981)). Als Linkerbaustein zur Verbindung zur herkömmlichen in Schema I synthetisierten PNA Synthese kann z.B. folgende Verbindung der allgemeinen Formel CN-X-NPG dienen: 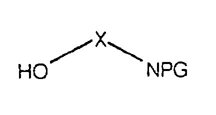 wobei X substituiertes Alkyl, Cycloalkyl, (Cyclo-)Alkenyl, (Cyclo-)Alkinyl, Aroyl, Heteroaroyl oder Heterocyclus bedeutet,
PG eine Schutzgruppe ist, die mit einem synthetisierten DNA oder RNA oder sonstigem Antisense Polymer Strang kompatibel sein muß, und z.B. Dimethoxytrityl, Fmoc, Moc, 5-Dimethoxybenzylcarbamat, oder o- und m-Nitrophenylcarbamate wie Nvoc oder 3,4-Dimethoxy-6-nitrobenzylcarbamat oder Phenyl(o-nitrophenyl)methylcarbamat bedeutet, wobei die Hydroxygruppe als Anker für die folgende RNA/DNA oder Phosphothioat Antisense Synthese dient.

Flüssigphasenverfahren werden bevorzugt zum Aufbau kürzerer aber großer Mengen von Oligomeren herangezogen, während Festphasenverfahren für den Aufbau langer und kleiner bis großer Mengen von Oligomeren geeignet sind und Oberflächenverfahren für den Aufbau langer und kleiner Mengen von Oligomeren geeignet sind.

### Experimentelle Bedingungen der Flüssig-Phasen Synthese

In flüssiger Phase wird die Aminkomponente mit der Oxokomponente, der Säurekomponente und einer geeignet substituierten amingeschützten Isocyanoaminkomponente entsprechend dem Formelschema I umgesetzt. Vorteilhaft werden dabei je ein Äquivalent der vier verschiedenen Komponenten zueinandergegeben und damit zur Reaktion gebracht. Vorteilhaft ist auch die Amin und die Oxokomponenten zur Schiff'schen Base vorzukondensieren. Als Lösungsmittel eigenen sich aprotisch polar wie unpolare sowie protisch polare. Aufgrund der Löslichkeitseigenschaften der Nucleobasensäurekomponenten eignen sich v.a. protische wie protisch polare Lösungsmittel wie z.B. Alkohole wie Wasser, Methanol, Ethanol, Propanol, Ethylenglycol, Glycerin, Trifluorethanol und aprotisch polare Lösungsmittel wie z.B. Pyridin, N-Methylmorpholin, Methylenchlorid, Chloroform, Dimethylformamid, Dimethylsulfoxid, Acetonitril, Ethylenglycoldimethylether oder auch Mischungen derselbigen wie z.B. Ethylenglycoldimethylether/Glycerin oder die Schiffbasenbildung fördernde Lösungsmittel wie Trimethylorthoformiat. Acylierungskatalysatoren wie z.B. Pyridin oder 4-Dimethylaminopyridin erweisen sich als reaktionsfördernd bei der Ugi Reaktion. Auch Lewis Säuren wie ZnCl₂, TiCl₄, ZrCp₂Cl₂ u.s.w. erweisen sich als reaktionsfördernd bei der Ugi Reaktion. Die Reaktionstemperatur kann -20°C und + 100°C, bevorzugt jedoch 10-40°Cbetragen. Die Reaktionszeit beträgt je nach Reaktivität der Komponenten Sekunden bis mehrere Tage. Vorteilhaft wird die Ugi Reaktion konzentriert durchgeführt, d.h. die Konzentrationen der Einzelkomponenten betragen 0.1M bis 4M.

### Experimentelle Bedingungen der Fest-Phasen Synthese

Polymere für die Fest-Phasen Synthese von Nucleobasenpolymeren können z.B. Polystyrol, Polyethylenglycol, Polyethylenglycol-Polystyrol Copolymere, Polyacrylamid, Controled Porous Glass (CPG), Teflon, Polyethylen, Polypropylen, Nylon, Cellulose sein. Geeignete Linker sind z.B. Aminomethyl, 4-Methylbenzhydrylamin (MBHA), 4-(2',4'-Dimethyloxyphenyl-Fmoc-aminomethyl)-phenoxyacetamido-norleucylaminomethyl (Rink Amide AM), 4-(2',4'-Dimethyloxyphenyl-Fmoc-aminomethyl)-phenoxyacetamido-norleucyl-MBHA (Rink Amide MBAH), 4-(2',4'-Dimethyloxyphenyl-Fmoc-aminomethyl)-phenoxy (Rink Amid), 9-Fmoc-amino-xanthen-3-yloxy (Sieber Amid), Hydrazine-2-chlorotrityl, 4-Sulfamylbenzoyl AM, 4-Sulfamylbutyryl AM, Bis-(2-aminoethyl)-ethertrityl, 1,3-Bis-(aminomethyl-phenyltrityl, oder mit N-terminal geschützten Aminosäuren beladene Harze.

Die Edukte der Synthese können in verschiedensten im vorhergehenden Abschnitt genannten, mit den Quelleigenschaften der Polymere kompatiblen Lösungsmitteln vorliegen. Bezogen auf die Beladung des Polmer werden die Edukte im 2 - 20 fachen Überschuss eingesetzt. Auch Lewis Säuren wie ZnCl₂, TiCl₄, ZrCp₂Cl₂ u.s.w. erweisen sich als reaktionsfördernd bei der Ugi Reaktion. Die Reaktionstemperatur kann -20°C und + 100°C, bevorzugt jedoch 10-40°C betragen. Die Reaktionszeit beträgt je nach Reaktivität der Komponenten Sekunden bis mehrere Tage. Vorteilhaft wird die Ugi Reaktion konzentriert durchgeführt, d.h. die Konzentrationen der Einzelkomponenten betragen 0.1M bis 4M.

### Experimentelle Bedingungen der Synthese an Oberflächen

Die Oberflächen müssen durch Linker für die Polymersynthese modifiziert werden. Mögliche Oberflächen sind z.B. Glasoberflächen, Polymeroberflächen wie Teflon, Polyethylen, Polypropylen, Nylon, Cellulose. Vorteilhaft werden über einen Linker Photoschutzgruppen auf der Oberfläche angebracht. Durch Entfernung der Schutzgruppen eines selektiven Bereiches der Oberfläche kann mit dem in Schema I beschrieben Verfahren zum Aufbau von PNAs begonnen werden. Vorteilhaft sind alle Schutzgruppen der einzelnen Komponenten Photoschutzgruppen wie in nachfolgender Abbildung gezeigt.

### Vorteile und mögliche Anwendungen der beschriebenen Erfindung

Das hier vorgestellte Verfahren zur Herstellung von Polymeren mit Nucleobasen als Seitengruppen unter Anwendung von Multikomponenten Reaktionen übertrifft in der Syntheseeffektivität und der Zugänglichkeit neuer PNAs alle bisher beschriebenen Verfahren. Die bisher beschriebenen PNA Varianten besitzen viele Nachteile für eine Anwendung als Antisense und Antigene Wirkstoffe. Am effektivsten kann nach neuen verbesserten Varianten mit dem hier vorgestellten Verfahren gesucht werden. Da bei bisherigen Verfahren über mehrere Stufen herzustellende Monomere zu den PNAs polymerisiert wurden ist die Variationseffektivität verglichen mit dem hier vorgestellten Verfahren wesentlich geringer. Bei dem vorgestellten Verfahren wird eine Monomereinheit aus vier verschiedenen Komponenten in einem Schritt hergestellt. Da die Edukte der Ugi Reaktion in großer Vielfalt kommerziell erhältlich sind oder einfach in ein bis zwei Stufen aus kommerziell erhältlichen Verbndungen leicht zugänglichen sind können in der gleichen Zeit bei vergleichbarem Aufwand wesentlich mehr Varianten hergestellt werden.

über das hier vorgestellte kombinatorische Verfahren dargestellte Monomer oder Di- und Trimere können potente Virostatika oder Cancerostatika dargestellt werden.

### Beispiele

Die verwendeten Abkürzungen sind nachfolgend aufgelistet.
- Alloc: Allyloxycarbonyl
- Boc: tert.-Butyloxycarbonyl
- Boc₂O: Pyrokohlensäure-di-tert.-butylester
- DCM: Dichlormethan
- DMF: Dimethylformamid
- Dmt.: 4,4'-Dimethoxytriphenylmethyl
- Fmoc: 9-Fluorenylmethoxycarbonyl
- FmocONSu: N-(9-Fluorenylmethoxycarbonyloxy)-succinimid
- Mmt: 4-Methoxytriphenylmethyl
- Moz: p-Methoxybenzyloxycarbonyl
- Pixyl: 9-(9-Phenyl)xanthenyl
- TBDMS: tert.-Butyldimethylsilyl
- TFA: Trifluoressigsäure
- Trt: Triphenylmethyl
- Z: Benzyloxycarbonyl

### Allgemeine Vorschrift zur Synthese der 2-Tritylamino-ethylisocyanidreste (X)

### Darstellung der 2-Tritylethylendiamine

Zu 12.20 g (200 mmol) Ethylendiamin in 500 ml THF werden bei RT langsam 40 mmol des jeweiligen Tritylchlorides (Triphenylmethylchlorid, 4-Methoxytriphenylmethylchlorid, 4,4'-Dimethoxytriphenylmethylchlorid), welches in 500 ml THF gelöst wurde, getropft. Danach wird 12 h bei RT gerührt, das Lösungsmittel am Rotationsverdampfer entfernt, der ölige, leicht gelbe bis orange Rückstand in 500 ml Essigester aufgenommen und je dreimal mit 250 ml gesättigter Natriumchloridlösung extrahiert. Die organische Phase wird mit Natriumsulfat getrocknet. Nach Entfernen des Lösungsmittels wird das jeweilige Amin als Öl bzw. Schaum in Ausbeuten von 50-95% erhalten.

### N-Tritylethylendiamin

C₂₁H₂₂N₂ (288.42)
R_{F}= 0.21 (DCM/MeOH 5:1, v,v); Spot färbt mit Ninhydrinspray bzw. mit HCl-Dampf)

### N-(4-Monomethoxytrityl)ethylendiamin

C₂₂H₂₄N₂O (332.45)
R_{F}= 0.21 (DCM/MeOH 5:1, v,v); Spot färbt mit Ninhydrinspray bzw. mit HCl-Dampf)

### N-(4,4'-Dimethoxytrityl)ethylendiamin

C₂₃H₂₆N₂O₂ (362.48)
R_{F}= 0.22 (DCM/MeOH 5:1, v,v); Spot färbt mit Ninhydrinspray bzw. mit HCl-Dampf)

### Darstellung der 2-Tritylaminoethylformamide

190 mmol des jeweiligen Amins werden in 300 ml Ameisensäureethylester 12 h refluxiert (Ölbadtemperatur 75°C). Nach Entfernen des überschüssigen Esters am Rotationsverdampfer wird das jeweilige Formamid als stabiler bzw. klebriger, gelber bis oranger Schaum erhalten. Die Ausbeuten liegen zwischen 80 und 95%.

### 2-Tritylaminoethylformamid

C₂₂H₂₂N₂O (330.42)
R_{F}= 0.87 (DCM/MeOH 5:1, v,v); Spot färbt mit Ninhydrinspray bzw. mit HCl-Dampf)

### 2-(4-Monomethoxytrityl)aminoethylformamid

C₂₃H₂₄N₂O₂ (360.46)
R_{F}= 0.86 (DCM/MeOH 5:1, v,v); Spot färbt mit Ninhydrinspray bzw. mit HCl-Dampf)
¹H-NMR (CDCl₃, 250.133 MHz): = 1.70 (br s, 1H); 2.32 (tr, 2H, J = 6.0 Hz); 3.37 (m, 2H); 3.76 (s, 3H); 5.99 (br s, 1H); 6.78-7.49 (m, 14H); 8.17 (s, 1H).
¹³C-NMR (CDCl₃, 62.896 MHz):
= 38.7 (CH₂); 43.1 (CH₂); 55.2 (CH₃); 70.2 (C); 113.2 (CH); 126.4 (CH); 127.9 (CH); 128.3 (CH); 129.7 (CH); 137.8 (C); 145.9 (C); 158.0 (C); 164.7 (CHO).

### 2-(4,4'-Dimethyoxytrityl)aminoethylformamid

C₂₄H₂₆N₂O₃ (390.49)
R_{F}= 0.92 (DCM/MeOH 5:1, v,v); Spot färbt mit Ninhydrinspray bzw. mit HCl)

### Darstellung der 2-Tritylaminoethylisonitrile

180 mmol des jeweiligen Formamides werden in 250 ml Methylenchlorid gelöst. Nach Zugabe von 400 mmol TEA kühlt man auf 0°C, tropft langsam 180 mmol Phosphoroxychlorid zu und läßt weiter bei dieser Temperatur zwei Stunden rühren. Dann werden bei 20°C 340 mmol Natriumcarbonat in 150 ml Wasser langsam unter starkem Rühren hinzugefügt und es wird noch 30 min bei RT nachgerührt. Die wäßrige Phase wird auf 400 ml verdünnt und zweimal mit je 150 ml DCM extrahiert. Nach Waschen der organischen Phase mit gesättigter NaCl-Lösung, Trocknen über Kaliumcarbonat und Abziehen des Lösungsmittels resultieren die jeweiligen Isonitrile als Schaum in 85-95% Ausbeute.

### 2-Tritylaminoethylisocyanid

C₂₂H₂₀N₂ (312.42)
R_{F}= 0.90 (DCM/MeOH 5:1, v,v); Spot färbt mit Ninhydrinspray bzw. mit HCl-Dampf)

### 2-(4-Monomethoxytrityl)aminoethylisocyanid

C₂₃H₂₂N₂O (342.44)
R_{F}= 0.89 (DCM/MeOH 5:1, v,v); Spot färbt mit Ninhydrinspray bzw. mit HCl-Dampf)

### 2-(4,4'-Dimethyoxytrityl)aminoethylisocyanid

C₂₄H₂₄N₂O₂ (372.47)
R_{F}= 0.95 (DCM/MeOH 5:1, v,v); Spot färbt mit Ninhydrinspray bzw. mit HCl-Dampf)

### Allgemeine Vorschrift zur Synthese der N-Acylethylisocyanide

### N-(tert.-Butyloxycarbonyl)ethylendiamin

Zu einer Lösung von 2.0 mol Ethylendiamin in 700 ml THF werden bei RT 0.25 mol Boc₂O in 500 ml THF innerhalb von 12-48 h getropft. Die Lösung wird vom ausgefallenen Feststoff abdekantiert und das Lösungsmittel abgezogen. Der Rückstand und der ausgefallene Feststoff werden in 500 ml Wasser gelöst und die entstandene Suspension filtriert. Die wäßrige Phase wird dreimal mit je 150 ml DCM extrahiert, mit konzentrierter NaCl-Lösung gewaschen, getrocknet und einrotiert. Es resultieren 36 g eines Öls (90% bez. auf Boc₂O).

### N-(tert.-Butyloxycarbonyl)aminoethylformamid

21.8 mmol N-(tert.-Butyloxycarbonyl)ethylendiamin werden in 50 ml Ameisensäureethyl- oder methylester gelöst und p-Toluolsulfonsäure als Katalysator zugegeben. Diese Mischung wird 12 h lang refluxiert. Nach Einrotieren der Lösung wird der Rückstand in 100 ml DCM oder Essigester aufgenommen, zweimal mit Wasser gewaschen, getrocknet und das Lösungsmittel abgezogen. Es werden 3.86 g eines Öls erhalten, das allmählich auskristallisiert (94% Ausbeute).
Fp.: 84-86°C

### 2-(N-tert.-Butyloxycarbonyl)aminoethylisonitril

Die Darstellung des Isonitrils erfolgt nach o.g. Vorschrift.
Es resultiert nahezu quantitativ ein Öl, das allmählich auskristallisiert.
¹H-NMR (250 MHz, CDCl₃): 1.42 (s, 9H, CH₃), 2.92 (m, 2H, CH₂), 3.40 (m, 2H, CH₂) 5.00 (s, 1H, NH).
¹³C-NMR (62 MHz, CDCl₃): 28.1 (CH₃); 38.1 (t, ¹J=7.5Hz, CH₂-NC), 39.0 (CH₂-NH), 80.0 (C), 155.8 (CO), 156.2 (t, ¹J=5.5Hz, NC).

Nach obigen Vorschriften werden folgende Verbindungen in analoger Weise hergestellt:

### 3-(N-tert.-Butyloxycarbonyl)aminopropylisonitril

¹H-NMR (250 MHz, CDCl₃): 1.44 (s, 9H, CH₃), 1.89 (m, 2H, CH₂), 3.26 (q, 2H, ³J=6.2Hz, CH₂), 3.46 (m, 2H, CH₂) 4.92 (s, 1H, NH). ¹³C-NMR (62 MHz, CDCl₃): 28.2 (CH₃); 29.4 (CH₂), 38.1 (t, ¹J=7.6Hz, CH₂-NC), 39.0 (CH-NH), 79.4 (C), 155,9 (CO), 156.4 (t, ¹J=5.3Hz, NC).

### 6-(N-tert.-Butyloxycarbonyl)aminohexylisonitril

¹H-NMR (250 MHz, CDCl₃): 1.36 (m, 6H, CH₂), 1.44 (s, 9H, CH₃), 1.89 (m ,2H, CH₂), 3.27 (m, 2H, CH₂), 3.47 (m, 2H, CH₂), 5.02, 4.92 (2s, 1H, NHCO-Rotamere).
¹³C-NMR (62 MHz, CDCl₃): 28.2 (CH₃), 28.3 (CH₂), 29.5 (CH₂), 30.5 (CH₂), 38.2 (t, ²J=6.7Hz, CH₂-NC), 39.0 (CH₂-NH), 77.4 (C), 156.2 (CO), 156.4 (t, ²J=5.3Hz, NC).

### 8-(N-tert.-Butyloxycarbonyl)aminooctylisonitril

¹H-NMR (250 MHz, CDCl₃): 1.31 (m, 10H, CH₂), 1.44 (s, 9H, CH₃), 1.69 (m ,2H, CH₂), 3.11 (m, 2H, CH₂-NC), 3.37 (m, 2H, CH₂-NHCO), 4.57 (s, 1H, NH).
¹³C-NMR (62 MHz, CDCl₃): 26.5 (CH₂), 26.6 (CH₂), 28.5 (CH₃), 28.9 (CH₂), 29.0 (CH₂), 29.1 (CH₂), 29.9 (CH₂), 40.5 (CH₂-NC), 41.5 (CH₂-NH), 77.4 (C), 156.6 (CO), 155.9 (NC).

### N-1-(4-Methoxybenzyloxycarbonyl)-N'-2-formylethylendiamin:

Synthese von *N*-(4-Methoxybenzyloxycarbonyl)-ethylendiamin nach Vorschrift [L.S.Richter, R.N. Zuckermann, *Bioorg. Med. Chem. Lett*., **5**, 1159-1162 (1995)], wobei die organische Methylenchloridphase 2 x mit 100 ml gesättigte Kochsalzlösung gewaschen wird. Ausbeute: 7.1 g, 64 % (Lit.: 6.0 g, 54 %).

7.1 g *N*-(4-Methoxybenzyloxycarbonyl)-ethylendiamin werden in 100 ml Ameisensäureethylester 3 Stunden und einer Spatelspitze 4-Dimethylaminopyridin unter Rückfluss gekocht. Der überschüssige Ameisensäureethylester wird am Rotationsverdampfer unter Vakuum abgezogen. Der verbleibende Feststoff wird in 100 ml CH₂Cl₂ aufgenommen und mit 30 ml H₂O ausgeschüttelt. Die organische Phase wird mit Na₂SO₄ getrocknet und unter Vakuum einrotiert. Das verbleibende Öl (6.53 g, 81 % Ausbeute) kristallisiert nach einiger Zeit und ist rein genug für die weitere Umsetzung.
¹H-NMR (250 MHz, CDCl₃): 3.37 (m, 4H), 3.80 (s, 3H), 5.02 (s, 2H), 5.28 (s, br, 1H), 6.32 (s, br, 1H), 6.87 (m, 2H), 7.27 (m, 2H), 8.14 (s, br, 1H).
¹³C-NMR (62 MHz, CDCl₃): 39,0, 40.6, 55.3, 66.8, 113.9, 128.4, 129.9, 159.7, 161.8, 163.4.
R_{f} (CH₂Cl₂/MeOH 9/1): 0.6

### N-1-(4-Methoxybenzyloxycarbonyl)-2-isocyano-1-aminoethan:

Zu 6.53 g *N*-1-(4-Methoxybenzyloxycarbonyl)-*N'*-2-formylethylendiamin (25.9 mmol) und 10.85 ml Triethylamin (78 mmol) in 100 ml CH₂Cl₂, temperiert mit einem Eisbad, werden langsam innerhalb von einer 1/2 h 3.97 g POCl₃ (25.91 mmol) getropft und das Gemisch weitere 4 h lang bei 0°C gerührt. Eine wässrige Lösung von 5.51 g Na₂CO₃ in 40 ml H₂O wird langsam bei 0°C zugetropft. Schließlich wird noch 1 h lang bei Raumtemperatur gerührt. Es wird mit weiteren 100 ml Wasser verdünnt, die organische Phase abgetrennt und die wässrige Phase 2 x mit 50 ml CH₂Cl₂ extrahiert. Die vereinigten organischen Phasen werden mit MgSO₄ getrocknet und unter Vakuum einrotiert. Es verbleiben 6.0 g (99 %) roter Feststoff, dessen Reinheit ausreichend für die nachfolgenden Reaktionen ist. Eine analytische Probe, umkristallisiert aus Ethylacetat, ergibt einen farblosen Feststoff.
¹H-NMR (250 MHz, CDCl₃): 3.43 (m, 2H), 3.53 (m, 2H), 3.80 (s, 3H), 5.04 (s, 2H), 5.50 (s, br, 1H), 6.88 (m, 2H), 7.28 (m, 2H).
¹³C-NMR (62 MHz, CDCl₃): 40.2, 41.8, 55.3, 66.9, 114.0, 128.2, 130.0, 156.4, 159.7.
R_{f} (CH₂Cl₂/MeOH 9/1): 0.8.

### Beispiel 1

1mmol Thyminessigsäure, lmmol Oxokomponente, 1mmol Aminkomponente und 1mmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird. 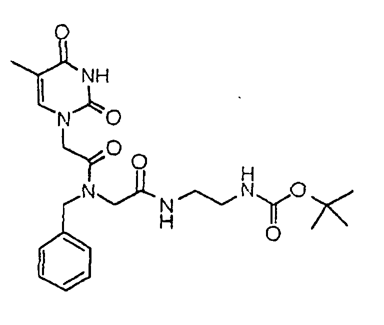 C₂₃H₃₁N₅O₆.
M_{w}: 473.53342
¹H-NMR (360 MHz / d₆-DMSO): 1.35 (9H, s), 1.76 (3H, s), 2.95 - 3.1 (4H, m), 3.77 and 3.93 (2H, 2 x s {rotamers}), 4.46, 4.58, 4.62, 4.67, 4.76, 4.80 (4H, 6 x s {rotamers}), 6.78 (1H, m), 7.21 - 7.39 (6H, m), 7.87 and 8.13 (1H, 2 x tr {rotamers}), 11.29 (1H, s, thymine-NH).
¹³C-NMR (62.9 MHz): 11.7, 28.1, 47.9, 48.8, 49.5, 77.5, 107.9, 127.1, 127.4, 127.6, 128.2, 128.5, 136.1, 136.8, 142.2, 150.9, 155.5, 158.0, 164.3, 167.2.
ES-MS: 474.0 (m + H)⁺

### Beispiel 2:

1mmol Thyminessigsäure, 1mmol Oxokomponente, 1mmol Aminkomponente und 1mmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird.

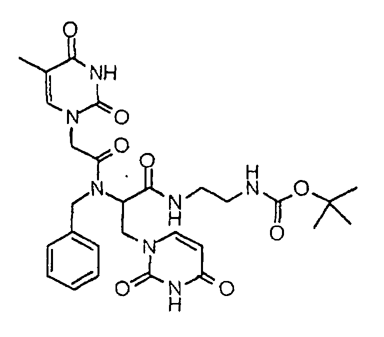

### Beispiel 3:

lmmol Thyminessigsäure, 1mmol Oxokomponente, lmmol Aminkomponente und lmmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird.

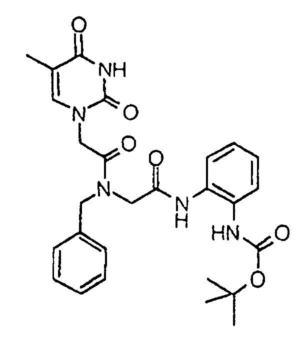

### Beispiel 4:

1mmol Thyminessigsäure, 1mmol Oxokomponente, 1mmol Aminkomponente und lmmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird.

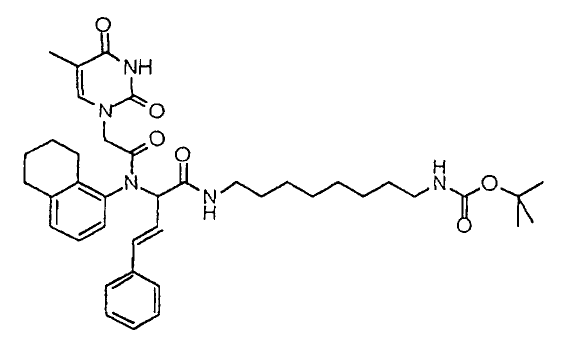

### Beispiel 5:

Beispiel 5:
Eine Deep Well Multi Titer Plate aus Polypropylene mit 1 ml Volumen je Vertiefung wird mit Thymin-1-essigsäure in Methanol und 1-Isocyano-2-N-tertbutoxycarbonylethan in Methanol je Vertiefung bestückt. Die Zeilen A - H werden mit den unten abgebildeten Aminen und die Spalten 1 - 12 mit den unten abgebildeten Oxokomponenten bestückt. Die verschlossenen MTP's werden bei Raumtemperatur geschüttelt und die ausgefallenen Produkte abfiltriert. Zur Erhöhung der Ausbeuten kann das Filtrat noch zur Hälfte eingeengt werden und die nochmals ausgefallenen Produkte abfiltriert werden. Die Produkte wurden durch HPLC-ES, DC und durch ¹H/¹³C-NMR charakterisiert.

### Bestückung der 96-MTP:

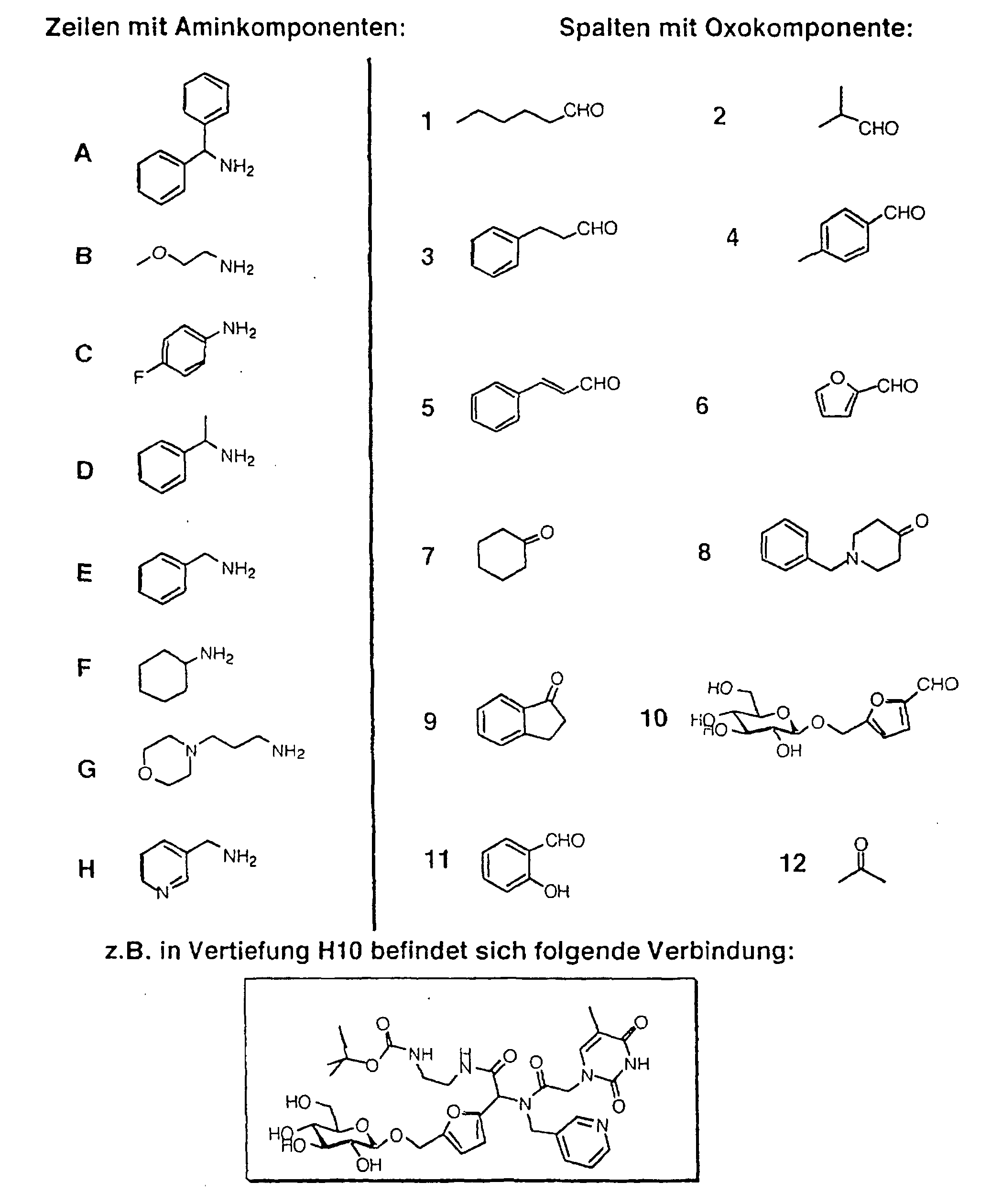

### Beispiel 6:

### Herstellung eines Thyminpentamer:

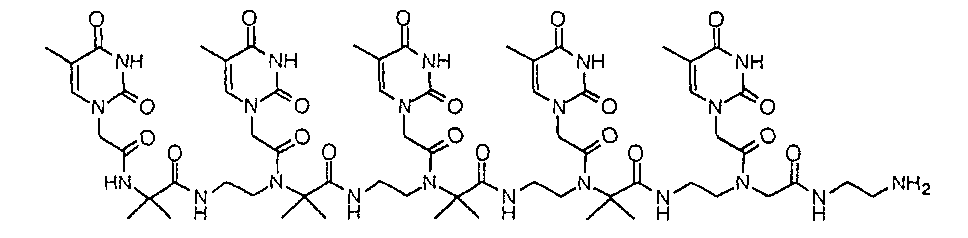

C₆₅H₉₃N₂₁O₂₀
Mw = 1488.56
HPLC-ESI-MS: entspicht

Zur Herstellung dieser Verbindung an Fmoc-Rinkamidharz wird nach folgendem Syntheseschema verfahren:
1. Entfernen der Fmoc-Gruppe des Rinkharz mit Morpholin.
2. Schütteln des so behandelten Harzes mit Aceton, Thyminessigsäure und N-1-(4-Methoxybenzyloxycarbonyl)-2-isocyano-1-aminoethan in DMSO/DMF.
3. Cappingschritt mit Essigsäureanhydrid in DMF.
4. Entfernen der Moz-Schutzgruppe mit 5% Trifluoressigsäure, 2.5% Thioanisol, 2.5% Dimercaptoethan in CH₂Cl₂.
5. Neutralisieren nit Morpholin.
6. Schritt 2-5 4 mal wiederholen.
7. Abspaltung vom Harz mit 95% TFA.

### Beispiel 7:

lmmol Thyminessigsäure, lmmol Oxokomponente, 1mmol Aminkomponente und lmmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird. 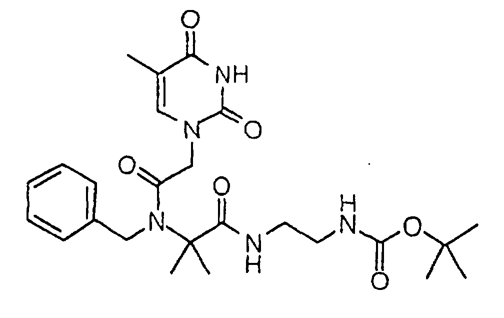 ¹H-NMR (250 MHz / d₆-DMSO): 1.23 (6H, s), 1.37 (9H, s), 1.75 (3H, s), 2.95 - 3.17 (4H, m), 4.58 (2H, s), 4.73 (2H, s), 6.70 (1H, tr), 7.29 - 7.50 (6H, m), 11.25 (1H, d, thymine-NH).
¹³C-NMR (62.9 MHz): 12.2, 24.4, 28.5, 43.0, 47.4, 49.7, 62.9, 78.0, 108.0, 126.9, 127.5, 128.9, 128.2, 138.8, 142.6, 151.4, 156.1, 164.8, 169.9, 174.1.
ES-MS: 502.0 (m + H)⁺

### Beispiel 8

1mmol Thyminessigsäure, lmmol Oxokomponente, 1mmol Aminkomponente und 1mmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird. 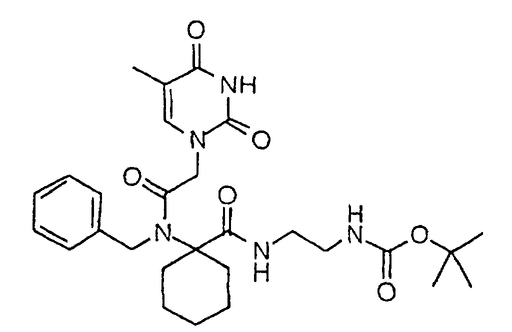 ¹H-NMR (360 MHz / d₆-DMSO): 0.83 - 1.54 (8H, m), 1.37 (9H, s), 1.75 (3H, s), 2.24 (2H, m), 2.95 - 3.17 (4H, m), 4.56 (2H, s), 4.73 (2H, s), 6.67 (1H, tr), 7.27 - 7.48 (6H, m), 11.28 (1H, s, thymine-NH).
¹³C-NMR (62.9 MHz): 11.6, 22.0, 24.7, 28.0, 32.2, 46.6, 49.7, 65.2, 77.4, 107.8, 126.5, 126.9, 128.4, 138.1, 136.1, 142.1, 142.4, 151.0, 155.5, 157.1, 164.3, 167.7, 172.7.
ES-MS: 542.0 (m + H)⁺

### Beispiel 9

1mmol Thyminessigsäure, 1mmol Oxokomponente, 1mmol Aminkomponente und lmmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird. 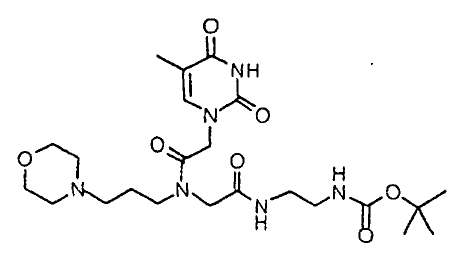 C₂₃H₄₈N₆O₇
M_{w}: 520.67501
¹H-NMR (250 MHz / d₆-DMSO):.
¹³C-NMR (62.9 MHz):11.8, 23.7, 24.0, 28.1, 44.9, 45.6, 47.9, 49.8, 53.0, 54.4, 55.3, 66.1, 77.6, 107.8, 142.3, 150.9, 155.5, 164.3, 166.9, 167.3, 167.9, 168.0.
ES-MS: 522 (m + H)⁺

### Beispiel 10:

Ausführung analog den vorhergehenden Beispielen.
Isocyanidkomponente: 1-Isocyano-3-N(tert.-Butoxycarbonyl)propan
Oxokomponente: Paraformaldehyd
Aminkomponente: Benzylamin
Säurekomponente: N-(p-Methoxybenzoyl)-N₉-adeninessigsäure
C₃₂H₃₈N₈O₆
Mw = 630.71
ES-MS: 632(m + H)⁺

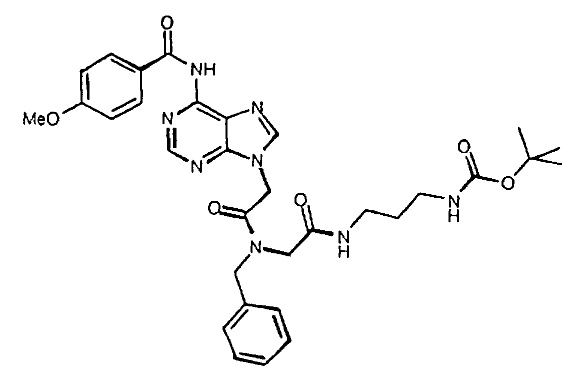

### Beispiel 11

1mmol Thyminessigsäure, 1mmol Oxokomponente, 1mmol Aminkomponente und 1mmol Isocyanidkomponente werden in 1 ml Methanol 24 h gerührt und das ausgefallene Produkt abfiltriert. Die Ausbeuten können erhöht werden, wenn das Filtrat zur Hälfte eingeengt wird und nochmals einer Filtration unterzogen wird. 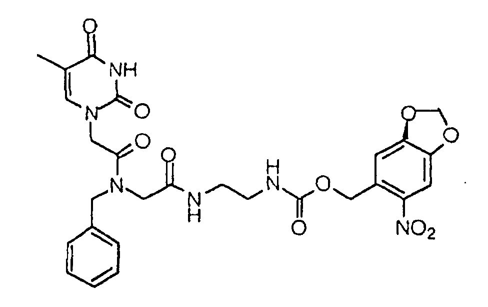 C₂₇H₂₈N₆O₁₀
Mw = 596.56

## Patentansprüche

1. Verfahren zur Herstellung von Verbindungen der Formel (I) 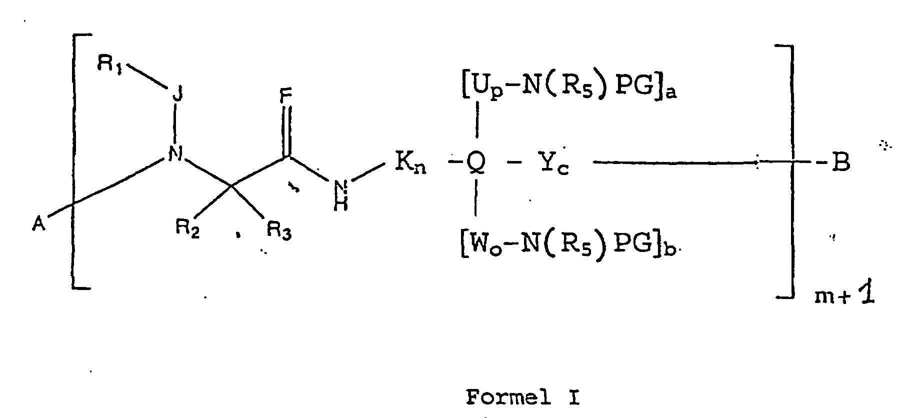 **dadurch gekennzeichnet, dass** Verbindungen der Formeln
A-NH₂ II
R₁-G III
R₂-C (O) -R₃ IV
und 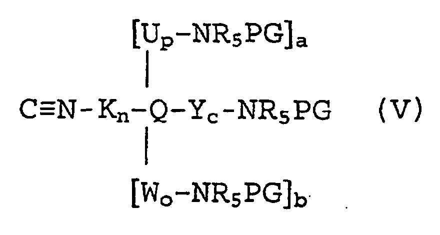 in einem ersten Schritt gegebenenfalls gleichzeitig miteinander umgesetzt werden,
gegebenenfalls eine oder mehrere Schutzgruppen entfernt werden und die Reaktion m-mal wiederholt wird,
wobei das Produkt des jeweils vorangegangenen Schrittes nach dem ersten Schritt anstelle der Verbindung mit der Formel II eingesetzt wird,
wobei
m 0 oder eine ganze Zahl von 1 bis 1000, vorzugsweise 1 bis 300 ist,
A
ein in der Ugi Reaktion üblicher Rest der Aminkomponente wie ein Wasserstoffatom, ein Substituent, (Cyclo-)Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein Saccharid oder Oligosaccharid, ein Antisensepolymer, ein Peptid, ein Antikörper-Konjugat, ein synthetisches Polymer oder eine durch Linker für die Polymersynthese modifizierte Oberfläche ist,
B
ein Wasserstoffatom, ein Substituent, (Cyclo-)Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein Saccharid oder Oligosaccharid, ein Antisensepolymer, ein Peptid, ein Antikörper-Konjugat, ein synthetisches Polymer oder eine durch Linker für die Polymersynthese modifizierte Oberfläche oder ein Rest 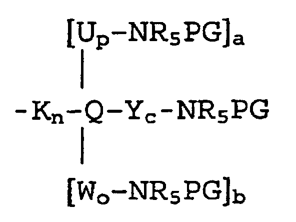
der Verbindung V ist,
R₁-G aus Strukturen der folgenden Formeln ausgewählt wird: 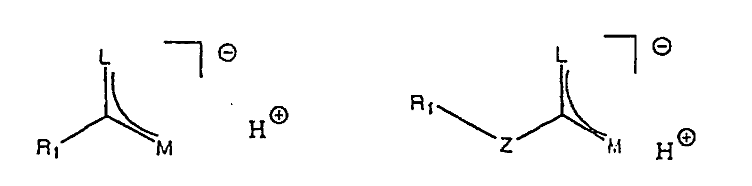 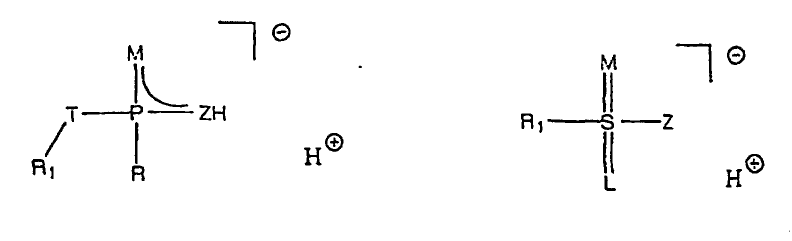 wobei die Gruppe R₁-G über einen molekularen Spacer über R₁ oder R oder R₄ mit der Verbindung der Formel IV verknüpft sein kann;
R₁ und R unabhängig voneinander ein in der Ugi Reaktion üblicher Rest der Säurekomponente wie H, ein Substituent, (Cyclo-)Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein Saccharid oder Oligosaccharid, ein Antisenspolymer, ein Peptid, ein Antikörper-Konjugat, ein synthetisches Polymer oder eine durch Linker für die Polymersynthese modifizierte Oberfläche, oder von den natürlichen Nucleobasen oder von synthetischen Nucleobasen abgeleitete Reste sind;
L, M, T und Z unabhängig voneinander O, S oder NR₄ bedeuten, wobei R₄ H, Fluor, (Cyclo-)Alkyl, (Cyclo-)Alkenyl, (Cyclo-)Alkinyl, Aroyl, Heteroaroyl, Heterocyclus oder - O-(Cyclo-)Alkyl, -O-Aroyl, -S-(Cyclo-)Alkyl, -S-Aroyl bedeuten;
R₂ und R₃ unabhängig voneinander ein in der Ugi Reaktion üblicher Rest der Oxokomponente wie H, ein Substituent, (Cyclo-)Alkyl, (Cyclo-)Alkenyl, (Cyclo-)Alkinyl, Aroyl, Heteroaroyl, ein Heterocyclus, ein Fluoreszenzmarker, ein Intercalator, ein Antibiotikum, ein Minor Groove Binder, ein Major Groove Binder, ein Biotinylrest, ein intercalierender Rest, ein alkylierender Rest, ein Steroid, ein Lipid, ein Polyamin, ein über einen Aminspacer verknüpftes Saccharid oder Oligosaccharid, ein Antisensemolekül, ein Peptid, ein Antikörper-Konjugat, ein synthetisches Polymer oder eine durch Linker für die Polymersynthese modifizierte Oberfläche sind,
PG unabhängig voneinander gegebenenfalls orthogonale Schutzgruppen wie Aminschutzgruppen aus der Klasse der N-Acylderivate, der N-Sulfonylderivate, der N-Alkylderivate, der N-Silylderivate, der Carbamate oder der Salze darstellen;
die Reste R₅ unabhängig voneinander Wasserstoffatome, unsubstituierte oder substituierte Alkyl-, Cycloalkyl-, Alkoxylalkyl- oder Arylgruppen oder Heterocyclen darstellen;
die Reste U, W, K und Y unabhängig voneinander unsubstituierte oder substituierte Alkyl-, Alkenyl-, Alkinyl-, Alkanoyl-, Alkoxyalkanoyl-, Cycloalkyl- oder Arylgruppen, unsubstituierte oder substituierte Heterocyclen oder die Gruppe NR₅ darstellen, wobei R₅ wie vorstehend definiert ist;
a, b, c, n, o und p unabhängig voneinander eine ganze Zahl von 0-10 vorzugsweise 0-5 sind;
Q eine unsubstituierte oder substituierte Alkyl-, Aryl-, Alkenyl-, Alkinyl-, oder Cycloalkyl-Gruppe, oder einen unsubstituierten oder substituierten Heterocyclus, oder eine Gruppe der Formel SO₂ darstellt,
F eine Oxo, Thio, Seleno oder Iminogruppe darstellt, und
J aus den folgenden Strukturen ausgewählt wird, wobei der obere vertikale Strich die Bindung zu R₁ und der untere vertikale Strich die Bindung zum Stickstoffatom der Hauptkette des Polymers in der allgemeinen Formel I darstellt: 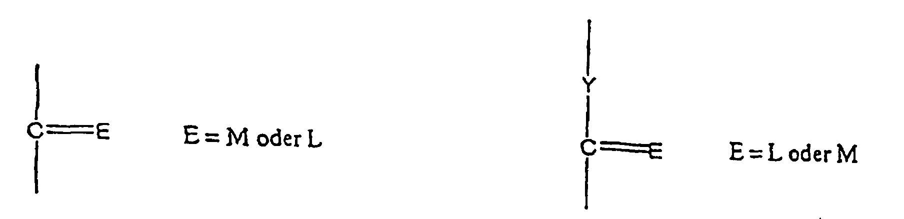 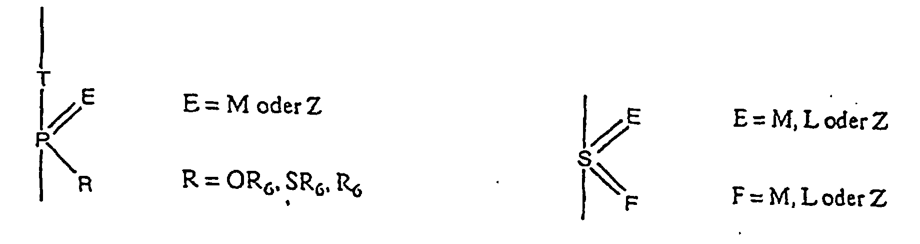 wobei R₆ H, einen Substituent, (Cyclo-)Alkyl, (Cyclo-)Alkenyl, (Cyclo-)Alkinyl, Aroyl, Heteroaroyl, Heterocyclus darstellt, mit der Maßgabe, dass mindestens einer der Reste R oder R₁ in der Verbindung der Formel I ein von einer natürlichen oder synthetischen Nucleobase abgeleiteter Rest ist, wobei alle vorstehenden Substituenten aus folgenden Gruppen ausgewählt werden:
- OH, -Rₐ, -O- (Cyclo)Alkyl, -O-Aryl, - O-Heteroaroyl, -O-Heterocyclus, -NH₂, -NO₂, -CN, -N₃, -CNRₐNR_{b}R_{c}, -NRₐR_{b}, NRₐR_{b}R_{c}⁺, Fluor, Chlor, Brom, α-β-, bis ω-Aminosäureester, -NRₐCOR_{b}, - NRₐCOXR_{b} (X = -O, -NR, -PO_{0,2,3,4}R, -SO_{0,1,2,4,}R, -NRₐNR_{b}R_{c}), -CORₐ, - COORₐ, -OCOORₐ, -CONRₐR_{b}, -OCONRₐR_{b}, -NR_{c} CONRₐR_{b}, -Rₐ-O-R_{b}, - R_{c}-NRₐR_{b}, -Rₐ-S-R_{b}, -Rₐ-SO-R_{b}, -Rₐ-S(O)₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, -NRₐCOR_{b}-O-R_{c}, -ORₐ-S-R_{b}, -NRₐR_{b}-S-R_{c}, - SO_{1,2,3,4}Rₐ-S-R_{b}, -CORₐ-S-R_{b}, -OCORₐ-S-R_{b}, NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, - CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b}, -NR_{c}POORₐOR_{b},
wobei Rₐ, R_{b}, Rₑ und R_{d} unabhängig voneinander wie oben definiert (Cyclo-)Alkyl, Alkenyl, Alkinyl, Aroyl, Heteroaroyl, ein Heterocyclus, Aralkyl, Aralkenyl oder Perhalogenalkyl sein können.

2. Verfahren nach Anspruch 1, **dadurch gekennzeichnet, dass** der Fluoreszenzmarker Fluorescein, Texas Rot, Lissamin Rhodamin, Cyanin oder Rhodamin, die Intercalatoren Psoralen, Acridin, Phenanthrolin, ein Phenanthrolin-Metall Komplex oder Ellipticin sind, die Antibiotika Endiine, β-Lactame, Tetracycline, Anthracycline, Polyether, Mitomiycin-artige, Phosphomycin-artige, Macrolide, Bleomycin-artige oder ein Aminoglycosid sind, der Minor Groove Binder Netropsin oder Distamycin ist, das Polyamin ein Spermidin-artiges Polyamin ist, das Antisensepolymer ein DNA- (5' oder 3' verknüpft) oder RNA Strang (5' oder 3' verknüpft) oder ein Phosphothioat ist, das Peptid N- oder C-terminal verknüpft ist, das Antikörper-Konjugat aus Antikörperkonjugaten ausgewählt wird, die zellspezifische Aufnahme gewähren, spezifische Carriersysteme ansprechen oder Endocytose bewirken, das synthetische Polymer CPG, Wang, oder Tentagel ist, die natürlichen Nucleobasen Adenin, Thymin, Guanin, Cytosin oder Uracil sind und die synthetischen Nucleobasen Pseudouracil, 5-Propinyluracil, 5-Hexenyluracil, 5-Fluorcytidin, 5-Hydroxymethyluracil, 5-Methylcytidin, 5-Bromcytidin und Verbindungen der folgenden Formel sind 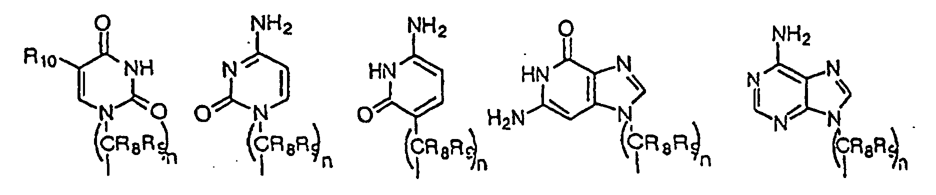 wobei R₈ und R₉ unabhängig voneinander H, Alkyl, (Cyclo-)Alkenyl, (Cyclo-)Alkinyl, Aroyl, Heteroaroyl, Heterocyclus, Chlor oder Fluor sind,
R₁₀ = Fluor, Brom, Jod, Chlor, Alkinyl, Alkyl, Aroyl, Heteroaroyl oder H ist, und n = 1 - 20, bevorzugt 1 - 10 und besonders bevorzugt 1 - 5 ist, wobei die Seitengruppen der Basen Schutzgruppen aufweisen können.

3. Verfahren nach einem der vorstehenden Ansprüche, **dadurch gekennzeichnet, dass** m = 1 - 200, vorzugsweise 1 - 100 ist.

4. Verfahren nach einem der vorstehenden Ansprüche, **dadurch gekennzeichnet, dass** in jedem Reaktionsschritt zumindest eine der Komponenten III, IV, und/oder V variiert wird.

5. Verfahren nach einem der vorstehenden Ansprüche, **dadurch gekennzeichnet, dass** es auf einer festen Phase wie einem Chip durchgeführt wird.

6. Verbindung der Formel V 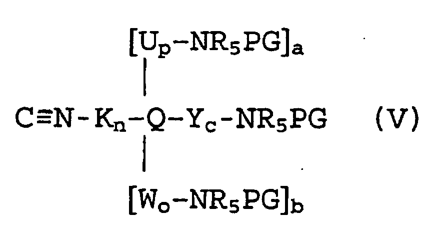 worin PG unabhängig voneinander gegebenenfalls orthogonale, an sich übliche, leicht abspaltbare (temporäre) Schutzgruppen für Amingruppen aus der Klasse der N-Silylderivate, darstellen oder aus der Gruppe ausgewählt sind: tert. Boc-, Alloc-, Fmoc-, Moz-, Z-, Tr-, MMTr-, DMTr-, Pixyl-, und TBDMS- Schutzgruppe;
die Reste R₅ unabhängig voneinander Wasserstoffatome, unsubstituierte oder substituierte Alkyl- Cycloalkyl-, Alkoxyalkyloder Arylgruppen oder Heterocyclen darstellen;
die Reste U, W, K und Y unabhängig voneinander unsubstituierte oder substituierte Alkyl-, Alkenyl-, Alkinyl-, Alkanoyl-, Alkoxyalkanoyl-, Cycloalkyl- oder Arylgruppen, unsubstituierte oder substituierte Heterocyclen oder die Gruppe NR₅ darstellen, wobei R₅ wie vorstehend definiert ist;
a, b, c, n, o und p unabhängig voneinander eine ganze Zahl von 0-10, vorzugsweise 0-5 sind;
Q eine unsubstituierte oder substituierte Alkyl-, Aryl-, Alkenyl-, Alkinyl-, oder Cycloalkyl-Gruppe, oder einen unsubstituierten oder substituierten Heterocyclus, oder eine Gruppe der Formel SO₂ darstellt,
wobei alle vorstehenden Substituenten aus folgenden Gruppen ausgewählt werden:
- OH, -Rₐ, -O- (Cyclo)Alkyl, -O-Aryl, - O-Heteroaroyl, -O-Heterocyclus, -NH₂, -NO₂, -CN, -N₃, -CNRₐNR_{b}R_{c}, -NRₐR_{b}, NRₐR_{b}R_{c}⁺, Fluor, Chlor, Brom, α-β-, bis ω-Aminosäureester, -NRₐCOR_{b}, -NRₐCOXR_{b} (X = -O, -NR, -PO_{0,2,3,4}R, -SO_{0,1,2,4,}R, -NRₐNR_{b}R_{c}), -CORₐ, - COORₐ, -OCOORₐ, -CONRₐR_{b}, -OCONRₐR_{b}, -NR_{c}CONRₐR_{b}, -Rₐ-O-R_{b}, - R_{c}-NRₐR_{b}, -Rₐ-S-R_{b}, -Rₐ-SO-R_{b}, -Rₐ-S(O)₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, - OCOORₐ-O-R_{b}, -NR_{b}CORₐ-O-R_{b}, -CONRₐR_{b}-O-R_{c}, -OCONRₐR_{b}-O-R_{c}, - NR_{c}CONRₐR_{b}-O-R_{d}, -NRₐCOR_{b}-O-R_{c}, -ORₐ-S-R_{b}, -NRₐR_{b}-S-R_{c}, - SO_{1,2,3,4}Rₐ-S-R_{b}, -CORₐ-S-R_{b}, -OCORₐ-S-R_{b}, NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, - CORₐ-NR_{b}R_{c}, -COORₐ-NR_{b}R_{c}, -OCORₐ-NR_{b}R_{c}, -OCOORₐ-NR_{b}R_{c}, - NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, - NRₐCOOR_{b}-NR_{c}R_{d}, -POORₐOR_{b}, -NR_{c}POORₐOR_{b},
wobei Rₐ, R_{b}, R_{c} und R_{d} unabhängig voneinander wie oben definiert (Cyclo-)Alkyl, Alkenyl, Akinyl, Aroyl, Heteroaroyl, ein Heterocyclus, Aralkyl, Aralkenyl oder Perhalogenalkyl sein können.

7. Verbindung nach Anspruch 6, **dadurch gekennzeichnet, dass** die Reste R₅ unabhängig voneinander Wasserstoffatome oder unsubstituierte oder substituierte Alkyl- oder Arylgruppen darstellen;
die Reste U, W, K und Y unabhängig voneinander unsubstituierte oder substituierte Alkyl-, Cycloalkyl- oder Arylgruppen darstellen;
a, b, c, n, o und p unabhängig voneinander eine ganze Zahl von 0-5 sind;
Q mindestens eine unsubstituierte Alkyl-, Aroyl- oder Arylgruppe oder einen unsubstituierten oder substituierten Heterocyclus darstellt,
wobei alle vorstehenden Substituenten aus folgenden Gruppen ausgewählt werden:
- OH, -Rₐ, -O- (Cyclo)Alkyl, -O-Aryl, - O-Heteroaroyl, -O-Heterocyclus, -NH₂, -NO₂, -CN, -N₃, -CNRₐNR_{b}R_{c}, -NRₐR_{b}, NRₐR_{b}R_{c}⁺, Fluor, Chlor, Brom, α-β-, bis ω-Aminosäureester, -NRₐCOR_{b}, - NRₐCOXR_{b} (X = -O, -NR, -PO_{0,2,3,4}R, -SO_{0,1,2,4,}R, -NRₐNR_{b}R_{c}), -CORₐ, - COORₐ, -OCOORₐ, -CONRₐR_{b}, -OCONRₐR_{b}, -NR_{c}CONRₐR_{b}, -Rₐ-O-R_{b}, - R_{c}-NRₐR_{b}, -Rₐ-S-R_{b}, -Rₐ-SO-R_{b}, -Rₐ-S(O)₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, - OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, -NRₐCOR_{b}-O-R_{c}, -ORₐ-S-R_{b}, -NRₐR_{b}-S-R_{c}, - SO_{1,2,3,4}Rₐ-S-R_{b}, -CORₐ-S-R_{b}, -OCORₐ-S-R_{b}, NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, - SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, - NRₐCONR_{b}R_{c}-NHR_{d}, -NRₐCOOR_{b}-NR_{c}R_{d}, -POORₐOR_{b}, -NR_{c}POORₐOR_{b},
wobei Rₐ, R_{b}, R_{c} und R_{d} unabhängig voneinander wie oben definiert (Cyclo-)Alkyl, Alkenyl, Akinyl, Aroyl, Heteroaroyl, ein Heterocyclus, Aralkyl, Aralkenyl oder Perhalongenalkyl sein können.

8. Verbindungen nach Anspruch 6, 7, die aus folgenden Strukturen ausgewählt sind:

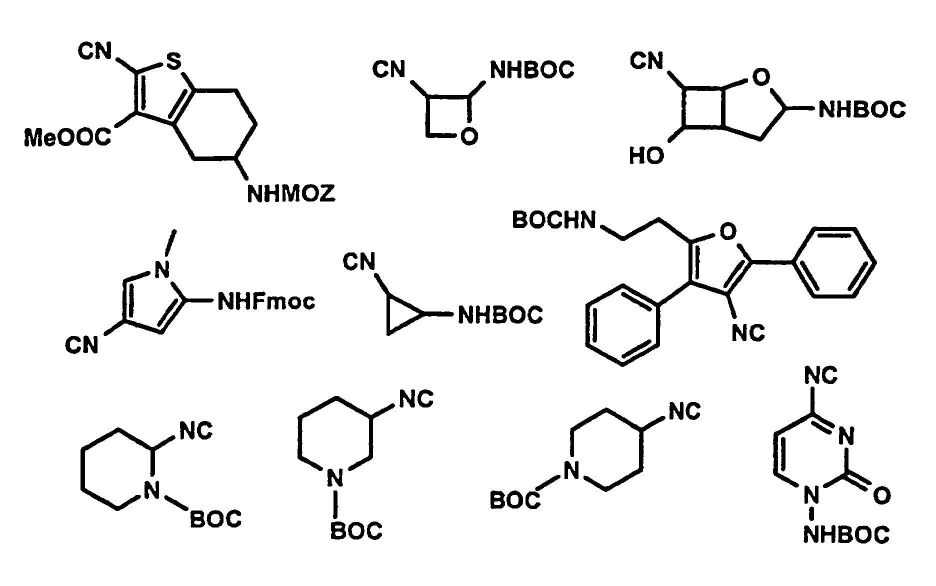

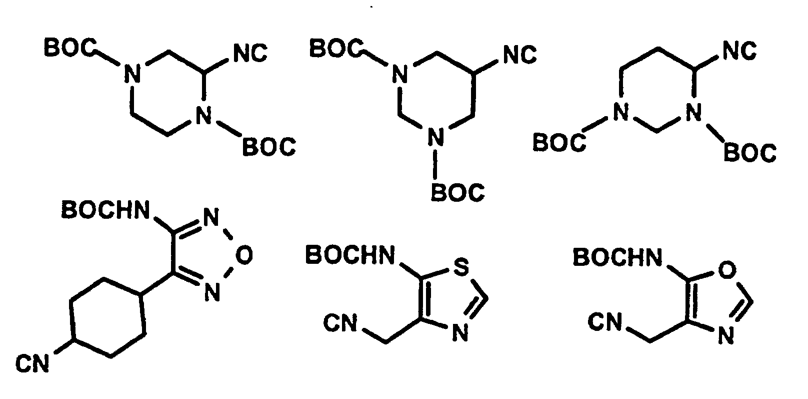

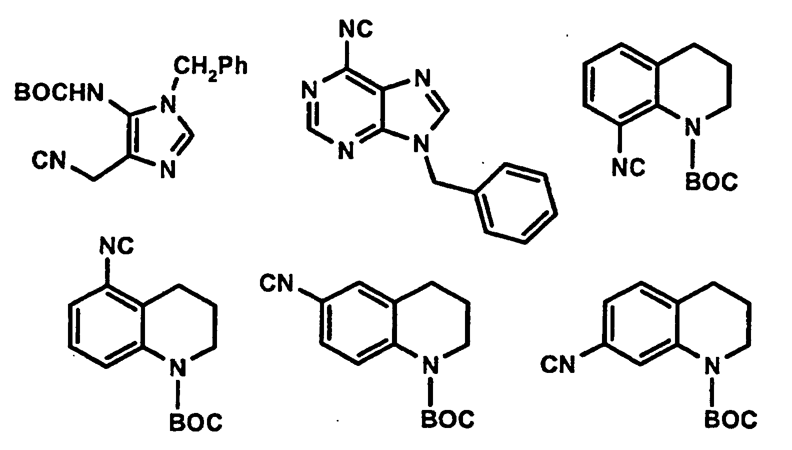

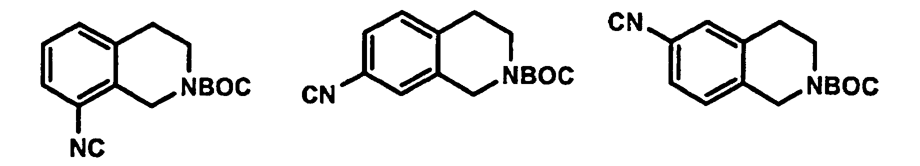

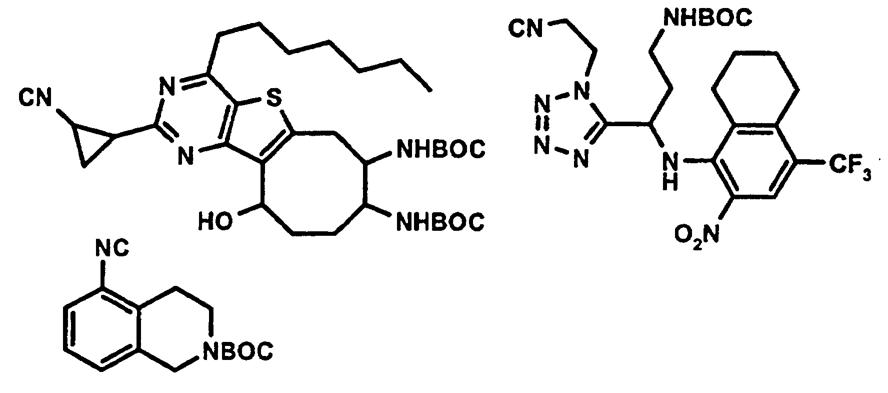

9. Verfahren zur Herstellung einer Verbindung der Formel V, **dadurch gekennzeichnet, dass** eine Verbindung der Formel VII 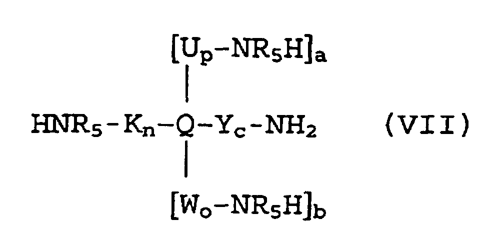 mit den wie in Anspruch 6 definierten Gruppen PG, die voneinander unabhängig sein können, mit üblichen Verfahren geschützt wird, wodurch eine Verbindung der Formel VIII hergestellt wird, 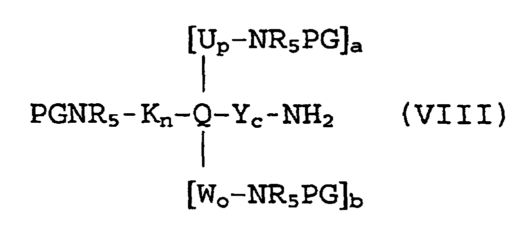 und dann die Verbindung der Formel VIII mit üblichen Verfahren zu einem Isonitril der Formel V umgesetzt wird.

10. Verfahren nach Anspruch 9, **dadurch gekennzeichnet, dass** die Verbindung der Formel VIII mit üblichen Formylierungsreagenzien zu einer Verbindung der Formel IX umgesetzt: 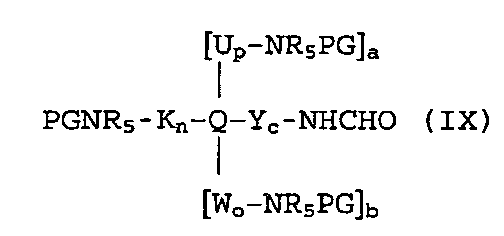 die dann unter üblichen Bedingungen zu einer Verbindung der Formel V umgesetzt wird, wobei die Reste und Indices wie in Anspruch 6 definiert sind.

11. Verfahren zur Herstellung einer Verbindung der Formel (IX) nach Anspruch 10, **dadurch gekennzeichnet, dass** man ausgehend von einer Verbindung der Formel (X) 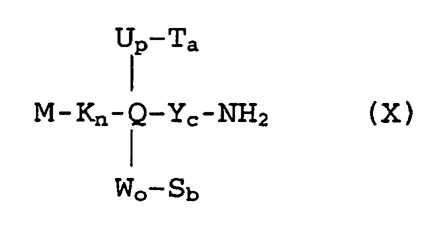 Wie in Anspruch 6 definiert, wobei die Reste und Indices wie vorstehend definiert sind, **dadurch gekennzeichnet, dass** zunächst die Aminfunktion formyliert, anschließend eine oder mehrere der Funktionalitäten M, T und S, die unabhängig voneinander Halogene oder Hydroxyfunktionen bedeuten, nach einem üblichen Verfahren in eine oder mehrere Azidfunktionen umgewandelt, diese Azide nach einem üblichen Verfahren in die entsprechenden Amine konvertiert und diese mit üblichen Verfahren mit o.g. Schutzgruppen versehen werden.

## Claims

1. Process for the preparation of compounds of formula (I) 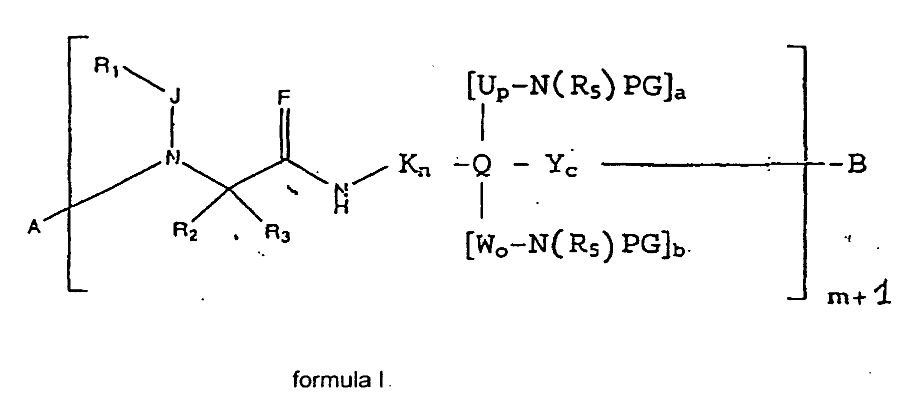 **characterised in that** compounds of formulae
A-NH₂ I
R₁-G II
R₂-C(O)-R₃ IV
and 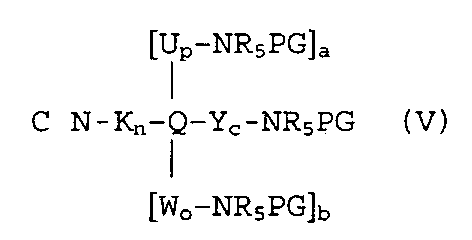 are reacted with one another, optionally simultaneously, in a first step,
as appropriate one or more protecting groups are removed,
and the reaction is repeated m times,
wherein, after the first step, the product of each preceding step is used instead of the compound of formula II,
wherein
m is 0 or an integer from 1 to 1000, preferably from 1 to 300,
A is a radical of the amine component being a radical customary in the Ugi reaction, such as a hydrogen atom, a substituent, (cyclo)alkyl, (cyclo)alkenyl, (cyclo)alkynyl, aroyl, heteroaroyl, a heterocycle, a fluorescent label, an intercalator, an antibiotic, a minor groove binder, a major groove binder, a biotinyl radical, an intercalating radical, an alkylating radical, a steroid, a lipid, a polyamine, a saccharide or oligosaccharide, an antisense polymer, a peptide, an antibody conjugate, a synthetic polymer or a surface modified by a linker for polymer synthesis,
B is a hydrogen atom, a substituent, (cyclo)alkyl, (cyclo)alkenyl, (cyclo)alkynyl, aroyl, heteroaroyl, a heterocycle, a fluorescent label, an intercalator, an antibiotic, a minor groove binder, a major groove binder, a biotinyl radical, an intercalating radical, an alkylating radical, a steroid, a lipid, a polyamine, a saccharide or oligosaccharide, an antisense polymer, a peptide, an antibody conjugate, a synthetic polymer or a surface modified by a linker for polymer synthesis or a radical 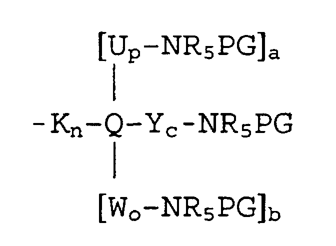 of compound V,
R₁-G is selected from structures of the following formulae: 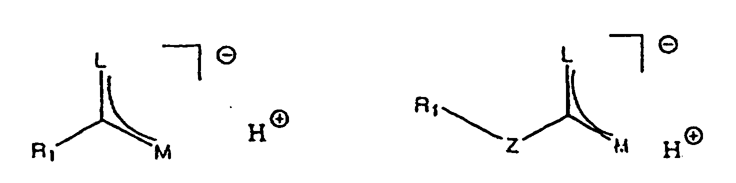 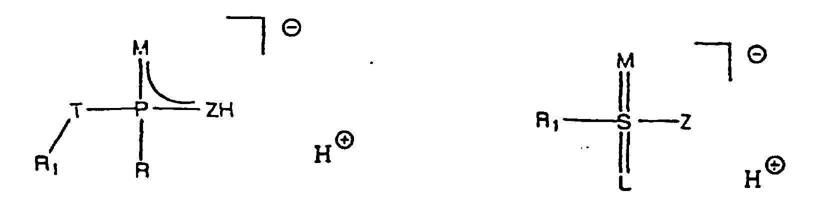 wherein the group R₁-G can be linked to the compound of formula IV by way of a molecular spacer *via* R₁ or R or R₄;
R₁ and R each independently of the other is a radical of the acid component being a radical customary in the Ugi reaction, such as H, a substituent, (cyclo)alkyl, (cyclo)alkenyl, (cyclo)alkynyl, aroyl, heteroaroyl, a heterocycle, a fluorescent label, an intercalator, an antibiotic, a minor groove binder, a major groove binder, a biotinyl radical, an intercalating radical, an alkylating radical, a steroid, a lipid, a polyamine, a saccharide or oligosaccharide, an antisense polymer, a peptide, an antibody conjugate, a synthetic polymer or a surface modified by a linker for polymer synthesis, or a radical derived from the natural nucleobases or from synthetic nucleobases;
L, M, T and Z each independently of the others represents O, S or NR₄, wherein R₄ represents H, fluorine, (cyclo)alkyl, (cyclo)alkenyl, (cyclo)alkynyl, aroyl, heteroaroyl, heterocycle or -O(cyclo)alkyl, -Oaroyl, -S(cyclo)alkyl, -Saroyl;
R₂ and R₃ each independently of the other represents a radical of the oxo component being a radical customary in the Ugi reaction, such as H, a substituent, (cyclo)alkyl, (cyclo)alkenyl, (cyclo)alkynyl, aroyl, heteroaroyl, a heterocycle, a fluorescent label, an intercalator, an antibiotic, a minor groove binder, a major groove binder, a biotinyl radical, an intercalating radical, an alkylating radical, a steroid, a lipid, a polyamine, a saccharide or oligosaccharide linked by way of an amine spacer, an antisense molecule, a peptide, an antibody conjugate, a synthetic polymer or a surface modified by a linker for polymer synthesis,
each PG independently of any others represents an optionally orthogonal protecting group such as an amine-protecting group from the class of N-acyl derivatives, N-sulphonyl derivatives, N-alkyl derivatives, N-silyl derivatives, carbamates or salts thereof;
each of the radicals R₅ independently of any others represents a hydrogen atom, an unsubstituted or substituted alkyl, cycloalkyl, alkoxyalkyl or aryl group or a heterocycle; the radicals U, W, K and Y each independently of the others represents an unsubstituted or substituted alkyl, alkenyl, alkynyl, alkanoyl, alkoxyalkanoyl, cycloalkyl or aryl group, an unsubstituted or substituted heterocycle or the group NR₅, wherein R₅ is as defined above;
a, b, c, n, o and p each independently of the others is an integer from 0 to 10, preferably from 0 to 5;
Q is an unsubstituted or substituted alkyl, aryl, alkenyl, alkynyl or cycloalkyl group, or an unsubstituted or substituted heterocycle, or a group of the formula SO₂,
F is an oxo, thio, seleno or imino group, and
J is selected from the following structures, wherein the upper vertical line represents the bond with R₁ and the lower vertical line represents the bond with the nitrogen atom of the main chain of the polymer in the general formula I: 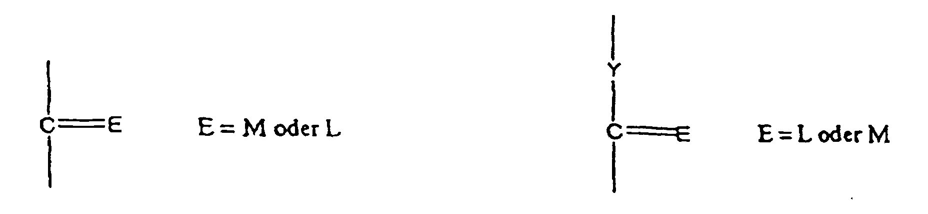 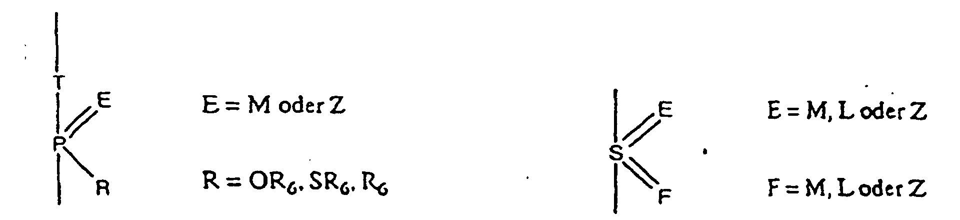 wherein R₆ is H, a substituent, (cyclo)alkyl, (cyclo)alkenyl, (cyclo)alkynyl, aroyl, heteroaroyl or heterocycle,
with the proviso that at least one of the radicals R and R₁ in the compound of formula I is a radical derived from a natural or synthetic nucleobase, wherein all substituents from above are selected from the following groups:
-OH, -Rₐ, -O-(cyclo)alkyl, -O-aryl, -O-heteroaroyl, -O-heterocycle, -NH₂, -NO₂, -CN, -N₃, -CNRₐNR_{b}R_{c}, -NRₐR_{b}, NRₐR_{b}R_{c}⁺, fluorine, chlorine, bromine, α-, β-, to ω-amino acid esters, -NRₐCOR_{b}, -NRₐCOXR_{b} (X = -O, -NR, -PO_{0, 2, 3, 4}R, -SO_{0, 1,2,4,}R, -NRₐNR_{b}R_{c}), -CORₐ, -COORₐ, -OCOORₐ, -CONRₐR_{b}, -OCONRₐR_{b}, -NR_{c}CONRₐR_{b}, -Rₐ-O-R_{b}, -R_{c}-NRₐR_{b}, -Rₐ-S-R_{b}, -Rₐ-SO-R_{b}, -Rₐ-S(O)₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, - NRₐCOR_{b}-O-R_{c}, -ORₐ-S-R_{b}, -NRₐR_{b}-S-R_{c}, -SO_{1,2,3,4}Rₐ-S-R_{b}, -CORₐ-S-R_{b}, -OCORₐ-S-R_{b}, NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b}, -NR_{c}POORₐOR_{b},
wherein R_{a,} R_{b}, R_{c} and R_{d} each independently of the others may be, as defined above, (cyclo)alkyl, alkenyl, alkynyl, aroyl, heteroaroyl, a heterocycle, aralkyl, aralkenyl or perhaloalkyl.

2. Process according to claim 1, **characterised in that** the fluorescent label is fluorescein, Texas red, lissamine rhodamine, cyanine or rhodamine, the intercalators are psoralen, acridine, phenanthroline, a phenanthroline/metal complex or ellipticine, the antibiotics are endiines, β-lactams, tetracyclins, anthracyclins, polyethers, mitomycin-like antibiotics, phosphomycin-like antibiotics, macrolides, bleomycin-like antibiotics or an aminoglycoside, the minor groove binder is netropsin or distamycin, the polyamine is a spermidine-like polyamine, the antisense polymer is a (5'- or 3'-linked) DNA strand or a (5'- or 3'-linked) RNA strand or a phosphothioate, the peptide is linked at the N- or C-terminal, the antibody conjugate is selected from antibody conjugates that provide cell-specific uptake, are responsive to specific carrier systems or bring about endocytosis, the synthetic polymer is CPG, Wang or Tentagel, the natural nucleobases are adenine, thymine, guanine, cytosine or uracil and the synthetic nucleobases are pseudouracil, 5-propynyluracil, 5-hexenyluracil, 5-fluorocytidine, 5-hydroxymethyluracil, 5-methylcytidine, 5-bromocytidine and compounds of the following formulae 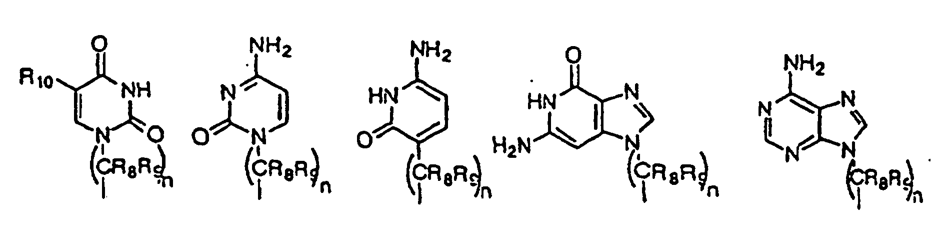 wherein R₈ and R₉ each independently of the other is H, alkyl, (cyclo)alkenyl, (cyclo)alkynyl, aroyl, heteroaroyl, heterocycle, chlorine or fluorine,
R₁₀ = fluorine, bromine, iodine, chlorine, alkynyl, alkyl, aroyl, heteroaroyl or H, and n = from 1 to 20, preferably from 1 to 10 and especially from 1 to 5, and wherein the side groups of the bases may have protecting groups.

3. Process according to any one of the preceding claims, **characterised in that**
m = from 1 to 200, preferably from 1 to 100.

4. Process according to any one of the preceding claims, **characterised in that** in each reaction step at least one of the components III, IV and/or V is varied.

5. Process according to any one of the preceding claims, **characterised in that** the process is carried out on a solid phase, such as a chip.

6. Compound of formula V 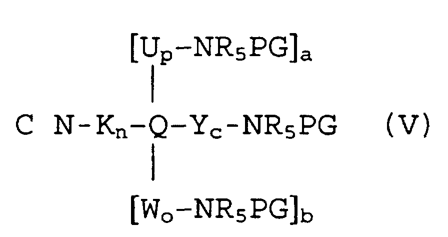 wherein each PG independently of any others represents an optionally orthogonal, customary, readily removable (temporary) protecting group for amine groups from the class of N-silyl derivatives or are selected from the group of protecting groups of tert.-Boc, Alloc, Fmoc, Moz, Z, Tr, MMTr, DMTr, Pixyl, and TBDMS;
each of the radicals R₅ independently of any others represents a hydrogen atom, an unsubstituted or substituted alkyl, cycloalkyl, alkoxyalkyl or aryl group or a heterocycle;
the radicals U, W, K and Y each independently of the others represents an unsubstituted or substituted alkyl, alkenyl, alkynyl, alkanoyl, alkoxyalkanoyl, cycloalkyl or aryl group, an unsubstituted or substituted heterocycle or the group NR₅, wherein R₅ is as defined above; a, b, c, n, o, and p each independently of the others is an integer from 0 to 10, preferably from 0 to 5;
Q is an unsubstituted or substituted alkyl, aryl, alkenyl, alkynyl or cycloalkyl group, or an unsubstituted or substituted heterocycle, or a group of the formula SO₂,
wherein all substituents from above are selected from the following groups:
- OH, -Rₐ, -O-(cyclo)alkyl, -O-aryl, -O-heteroaroyl, -O-heterocycle, -NH₂, -NO₂, -CN, -N₃, -CNRₐNR_{b}R_{c}, -NRₐR_{b}, NRₐR_{b}R_{c}⁺, fluorine, chlorine, bromine, α-, β-, to ω-amino acid esters, -NRₐCOR_{b}, -NRₐCOXR_{b} (X = -O, -NR, -PO_{0,2,3,4}R, -SO_{0,1,2,4,}R, -NRₐNR_{b}R_{c}), -CORₐ, -COORₐ, -OCOORₐ, -CONRₐR_{b}, -OCONRₐR_{b}, -NR_{c}CONRₐR_{b}, -Rₐ-O-R_{b}, - R_{c}-NRₐR_{b}, -Rₐ-S-R_{b}, -Rₐ-SO-R_{b}, -Rₐ-S(O)₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, - NRₐCOR_{b}-O-R_{c}, -ORₐ-S-R_{b}, -NRₐR_{b}-S-R_{c}, -SO_{1,2,3,4}Rₐ-S-R_{b}, -CORₐ-S-R_{b}, -OCORₐ-S-R_{b}, NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b}, -NR_{c}POORₐOR_{b},
wherein R_{a,} R_{b}, R_{c} and R_{d} each independently of the others may be, as defined above, (cyclo)alkyl, alkenyl, alkynyl, aroyl, heteroaroyl, a heterocycle, aralkyl, aralkenyl or perhaloalkyl.

7. Compound according to claim 6, **characterized in that**
each of the radicals R₅ independently of any others represents a hydrogen atom, an unsubstituted or substituted alkyl, cycloalkyl, alkoxyalkyl or aryl group or a heterocycle;
the radicals U, W, K and Y each independently of the others represents an unsubstituted or substituted alkyl, cycloalkyl or aryl group;
a, b, c, n, o, and p each independently of the others is an integer from 0 to 5;
Q is at least an unsubstituted alkyl, aroyl, aryl group or an unsubstituted or substituted heterocycle,
wherein all substituents from above are selected from the following groups:
- OH, -Rₐ, -O-(cyclo)alkyl, -O-aryl, -O-heteroaroyl, -O-heterocycle, -NH₂, -NO₂, -CN, -N₃, -CNRₐNR_{b}R_{c}, -NRₐR_{b}, NRₐR_{b}R_{c}⁺, fluorine, chlorine, bromine, α-, β-, to ω-amino acid esters, -NRₐCOR_{b}, -NRₐCOXR_{b} (X = -O, -NR, -PO_{0, 2, 3, 4}R, -SO_{0, 1, 2, 4,}R, -NRₐNR_{b}R_{c}), -CORₐ, -COORₐ, -OCOORₐ, -CONRₐR_{b}, -OCONRₐR_{b}, -NR_{c}CONRₐR_{b}, -Rₐ-O-R_{b}, - R_{c}-NRₐR_{b}, -Rₐ-S-R_{b}, -Rₐ-SO-R_{b}, -Rₐ-S(O)₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, - NRₐCOR_{b}-O-R_{c}, -ORₐ-S-R_{b}, -NRₐR_{b}-S-R_{c}, -SO_{1,2,3,4}Rₐ-S-R_{b}, -CORₐ-S-R_{b}, -OCORₐ-S-R_{b}, NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b}, -NR_{c}POORₐOR_{b},
wherein Rₐ, R_{b}, R_{c} and R_{d} each independently of the others may be, as defined above, (cyclo)alkyl, alkenyl, alkynyl, aroyl, heteroaroyl, a heterocycle, aralkyl, aralkenyl or perhaloalkyl.

8. Compounds according to claims 6 and 7 which are selected from the following structures:

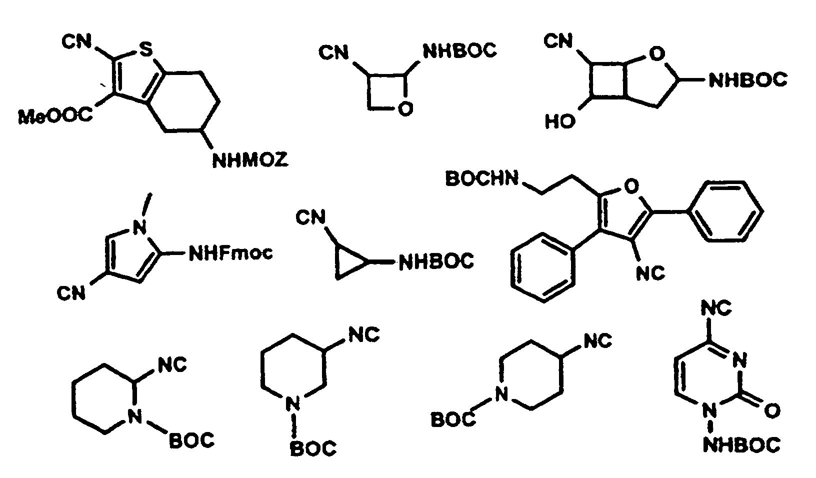

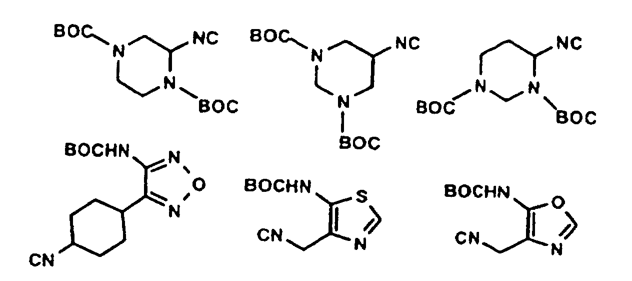

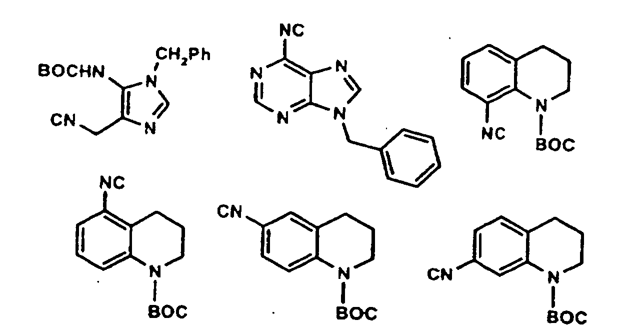

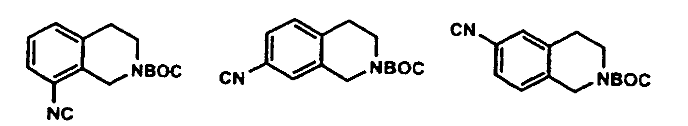

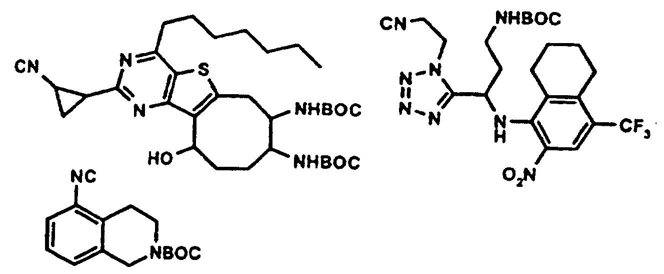

9. Process for the manufacture of a compound of formula V, **characterized in that** a compound of formula VII 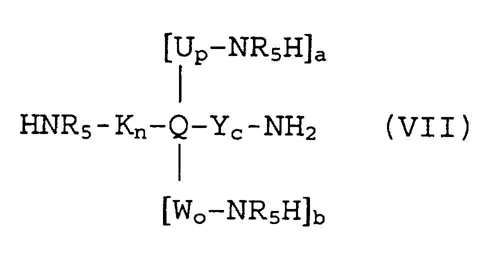 is protected with groups PG defined in claim 6, which may be independent of one another, by customary processes, resulting in a compound of formula VIII 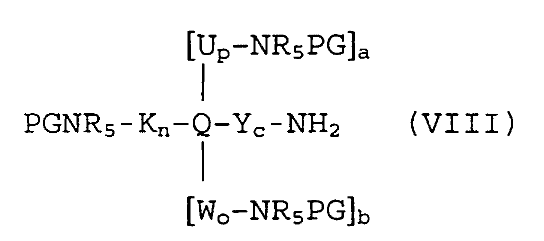 and then the compound of formula VIII is reacted by customary processes to form an isonitrile of formula V.

10. Process according to claim 9, **characterized in that**
the compound of formula VIII is reacted with customary formylation reagents to form a compound of formula IX: 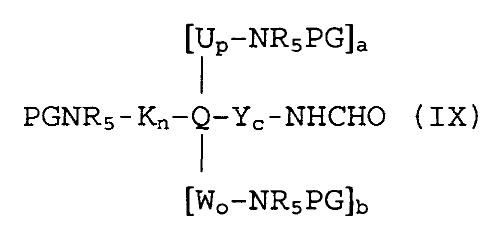 which is then reacted under customary conditions to form a compound of formula V, the radicals and indices being as defined in claim 6.

11. Process for the manufacture of a compound of formula (IX) according to claim 10, **characterized in that** starting from a compound of formula (X), 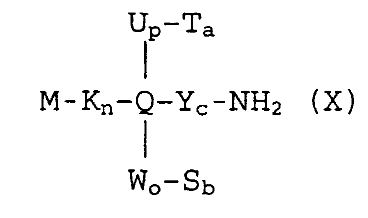 as defined in claim 6, wherein the radicals and indices are as defined above, **characterized in that** the amine function is formylated first, and then one or more of the functionalities M, T and S, which each independently of the others represents a halogen or a hydroxy function is/are converted into one or more azide functions according to a customary process, those azides are converted into the corresponding amines according to a customary process and the amines are then provided with above-mentioned protecting groups by customary processes.

## Revendications

1. Procédé de préparation de composés de formule I : 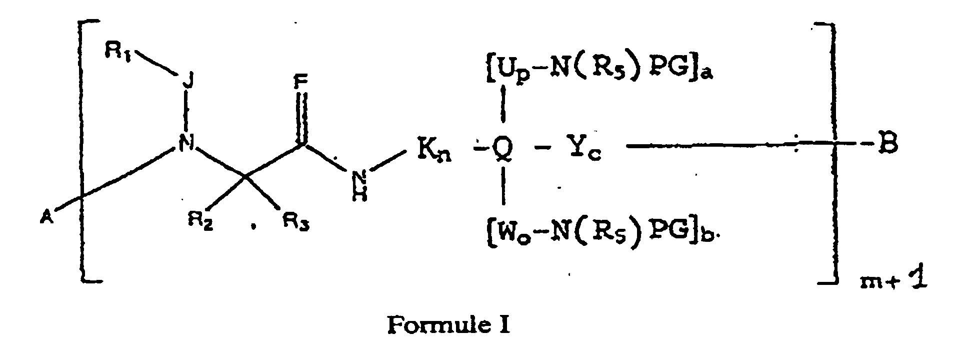 **caractérisé en ce que**, dans une première étape, on fait réagir les uns avec les autres, et simultanément le cas échéant, des composés de formules
A―NH₂ (II)
R₁―G (III)
R₂―C(O)―R₃ (IV)
et 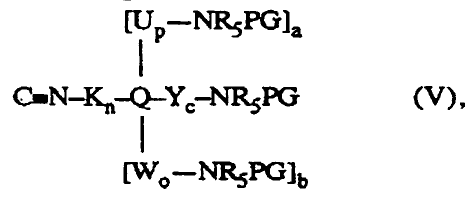 on élimine éventuellement un ou plusieurs groupes protecteurs, et l'on répète cette réaction m fois, mais en utilisant chaque fois après la première étape, à la place du composé de formule II, le produit de l'étape précédente respective ;
étant entendu que :
m vaut 0 ou est un nombre entier valant de 1 à 1000, et de préférence de 1 à 300 ;
A représente un reste de composant aminé habituel dans la réaction d'Ugi comme un atome d'hydrogène, un substituant, un groupe (cyclo)-alkyle, (cyclo)alcényle, (cyclo)alcynyle, aroyle ou hétéroaroyle, un reste hétérocyclique, un marqueur fluorescent, un agent intercalant, un antibiotique, un ligand du petit sillon, un ligand du grand sillon, un reste biotinyle, un reste intercalant, un reste alkylant, un stéroïde, un lipide, une polyamine, un saccharide ou un oligosacchacide, un polymère anti-sens, un peptide, un conjugué d'anticorps, un polymère synthétique, ou une surface modifiée par un élément de raccord pour synthèse de polymères ;
B représente un atome d'hydrogène, un substituant, un groupe (cyclo)alkyle, (cyclo)alcényle, (cyclo)alcynyle, aroyle ou hétéroaroyle, un reste hétérocyclique, un marqueur fluorescent, un agent intercalant, un antibiotique, un ligand du petit sillon, un ligand du grand sillon, un reste biotinyle, un reste intercalant, un reste alkylant, un stéroïde, un lipide, une polyamine, un saccharide ou un oligosaccharide, un polymère anti-sens, un peptide, un conjugué d'anticorps, un polymère synthétique, ou une surface modifiée par un élément de raccord pour synthèse de polymères, ou un reste 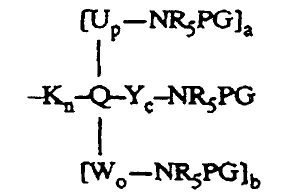 du composé de formule V ;
R₁―G est choisi parmi les structures de formules suivantes : 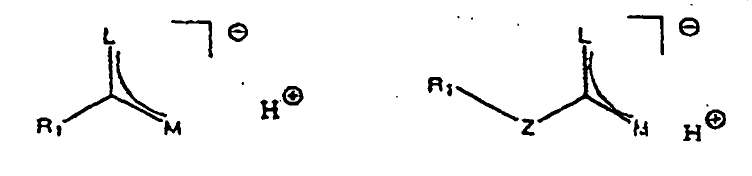 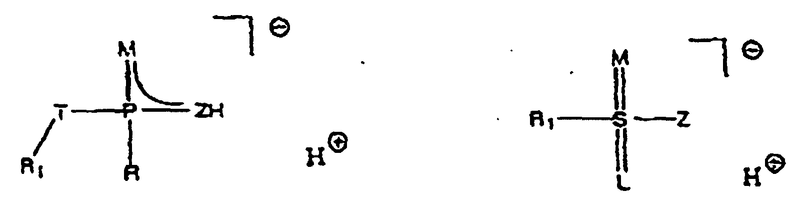 le groupe R₁―G pouvant être rattaché au composé de formule IV, par l'intermédiaire d'un espaceur moléculaire, par R₁, R ou R₄ ;
R₁ et R représentent chacun, indépendamment l'un de l'autre, un reste de composant acide habituel dans la réaction d'Ugi comme un atome d'hydrogène, un substituant, un groupe (cyclo)alkyle, (cyclo)alcényle, (cyclo)alcynyle, aroyle ou hétéroaroyle, un reste hétérocyclique, un marqueur fluorescent, un agent intercalant, un antibiotique, un ligand du petit sillon, un ligand du grand sillon, un reste biotinyle, un reste intercalant, un reste alkylant, un stéroïde, un lipide, une polyamine, un saccharide ou un oligosaccharide, un polymère anti-sens, un peptide, un conjugué d'anticorps, un polymère synthétique, ou une surface modifiée par un élément de raccord pour synthèse de polymères, ou des restes dérivés de bases nucléiques naturelles ou de bases nucléiques synthétiques ;
L, M, T et Z représentent chacun, indépendamment les uns des autres, O, S ou NR₄ où R₄ représente un atome d'hydrogène ou de fluor, un groupe (cyclo)alkyle, (cyclo)alcényle, (cyclo)alcynyle, aroyle ou hétéroaroyle, un reste hétérocyclique, ou un groupe -O-(cyclo)alkyle, -O-aroyle, -S-(cyclo)alkyle ou -S-aroyle ;
R₂ et R₃ représentent chacun, indépendamment l'un de l'autre, un reste de composant oxo habituel dans la réaction d'Ugi comme un atome d'hydrogène, un substituant, un groupe (cyclo)alkyle, (cyclo)alcényle, (cyclo)alcynyle, aroyle ou hétéroaroyle, un reste hétérocyclique, un marqueur fluorescent, un agent intercalant, un antibiotique, un ligand du petit sillon, un ligand du grand sillon, un reste biotinyle, un reste intercalant, un reste alkylant, un stéroïde, un lipide, une polyamine, un saccharide ou un oligosaccharide rattaché par un espaceur aminé, une molécule anti-sens, un peptide, un conjugué d'anticorps, un polymère synthétique, ou une surface modifiée par un élément de raccord pour synthèse de polymères ;
les PG représentent, indépendamment les uns des autres, des groupes protecteurs éventuellement orthogonaux, comme des groupes amino-protecteurs des classes des dérivés N-acyle, N-sulfonyle, N-alkyle ou N-silyle, des carbamates ou des sels ;
les R₅ représentent, indépendamment les uns des autres, des atomes d'hydrogène, des groupes alkyle, cycloalkyle, alcoxyalkyle ou aryle, substitués ou non substitués, ou des groupes hétérocycliques ;
les U, W, K et Y représentent, indépendamment les uns des autres, des groupes alkyle, alcényle, alcynyle, alcanoyle, alcoxyalcanoyle, cycloalkyle ou aryle, substitués ou non substitués, des groupes hétérocycliques substitués ou non substitués, ou des groupes NR₅ où R₅ a la signification indiquée précédemment ;
a, b, c, n, o et p représentent chacun, indépendamment les uns des autres, un nombre entier qui vaut de 0 à 10, et de préférence de 0 à 5 ;
Q représente un groupe alkyle, aryle, alcényle, alcynyle ou cycloalkyle, substitué ou non substitué, un groupe hétérocyclique substitué ou non substitué, ou un groupe de formule SO₂ ;
F représente un substituant oxo, thioxo, sélénoxo ou imino ;
et J est choisi parmi les structures suivantes, dans lesquelles le trait vertical supérieur représente la liaison vers R₁ et le trait vertical inférieur représente la liaison vers l'atome d'azote qui fait partie de la chaîne principale du polymère de formule générale I : 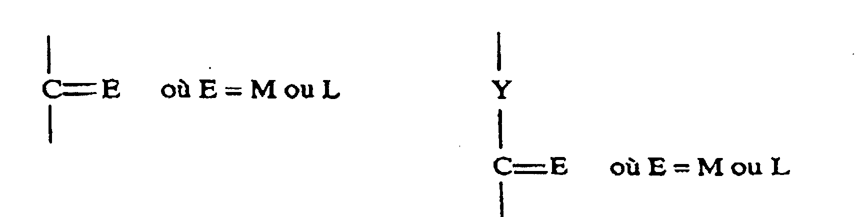 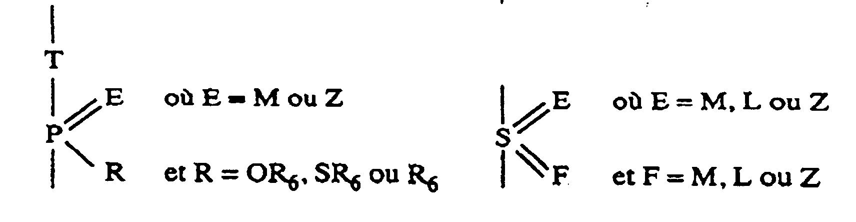 et où R₆ représente un atome d'hydrogène, un substituant, un groupe (cyclo)alkyle, (cyclo)alcényle, (cyclo)alcynyle, aroyle ou hétéroaroyle, ou un reste hétérocyclique, sous réserve qu'au moins l'un des restes représentés par R ou R₁ dans le composé de formule I soit un reste dérivé d'une base nucléique naturelle ou synthétique ;
tous les substituants mentionnés précédemment étant choisis dans l'ensemble suivant : -OH, -Rₐ, -O-(cyclo)alkyle, -O-aryle, -O-hétéroaroyle, -O-hétérocyclique, -NH₂, -NO₂, -CN, -N₃, -CNRₐNR_{b}R_{c}, -NRₐR_{b}, -N⁺RₐR_{b}R_{c}, fluoro, chloro, bromo, ester d'acide (alpha, bêta, ... oméga)-aminé, -NRₐCOR_{b}, -NRₐCOXR_{b} (où X représente -O, -NR, -PO_{0,2,3,4}R, -SO_{0,1,2,4}R ou -NRₐNR_{b}R_{c}), -CORₐ, -COORₐ, -OCOORₐ, -CONRₐR_{b}, -OCONRₐR_{b}, -NR_{c}CONRₐR_{b}, -Rₐ-O-R_{b}, -R_{c}-NRₐR_{b}, -Rₐ-S-R_{b}, -Rₐ-SO-R_{b}, -Rₐ-SO₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, -NRₐCOR_{b}-O-R_{c}, -ORₐ-S-R_{b}, -NRₐR_{b}-S-R_{c}, -SO_{1,2,3,4}Rₐ-S-R_{b}, -CORₐ-S-R_{b}, -OCORₐ-S-R_{b}, ―NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b} et -NR_{c}POORₐOR_{b}, où Rₐ, R_{b}, R_{c} et R_{d} peuvent représenter, indépendamment les uns des autres, des groupes (cyclo)alkyle, alcényle, alcynyle, aroyle, hétéroaroyle, hétérocycliques, aralkyle, aralcényle ou perhalogénoalkyle, tels que définis plus haut.

2. Procédé conforme à la revendication 1, **caractérisé en ce que** le marqueur fluorescent est la fluorescéine, le rouge Texas, la lissamine rhodamine, une cyanine ou une rhodamine, les agents intercalants sont le psoralène, l'acridine, la phénanthroline, un complexe métal-phénanthroline ou l'ellipticine, les antibiotiques sont des ènedyines, des β-lactarnes, des tétracyclines, des anthracyclines, des polyéthers, des macrolides, des composés du type de la mitomycine, de la phosphomycine ou de la bléomycine, ou des aminoglycosides, le ligand du petit sillon est la nétropsine ou la distamycine, la polyamine est une polyamine du type de la spermidine, le polymère anti-sens est un brin d'ADN lié en 5' ou en 3', un brin d'ARN lié en 5' ou en 3', ou un phosphorothioate, le peptide est lié par son extrémité amino ou carboxyle, le conjugué d'anticorps est choisi parmi les conjugués d'anticorps qui donnent une absorption spécifique de cellules, reconnaissent des systèmes vecteurs spécifiques ou provoquent une endocytose, le polymère synthétique est un support CPG, une résine Wang ou une résine Tenta-Gel, les bases nucléiques naturelles sont l'adénine, la thymine, la guanine, la cytosine et l'uracile, et les bases nucléiques synthétiques sont le pseudo-uracile, le 5-propynyl-uracile, le 5-hexényl-uracile, la 5-fluorocytidine, le 5-hydroxyméthyl-uracile, la 5-méthyl-cytidine, la 5-bromo-cytidine, et les composés de formules suivantes : 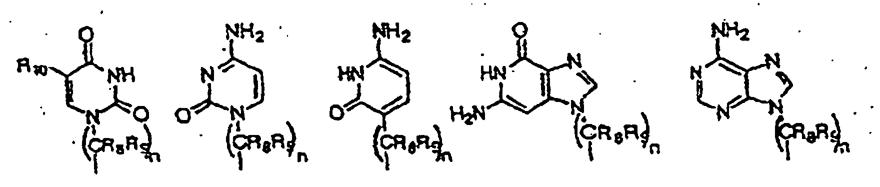 dans lesquelles R₈ et R₉ représentent chacun, indépendamment l'un de l'autre, un atome d'hydrogène, de chlore ou de fluor ou un groupe alkyle, (cyclo)alcényle, (cyclo)alcynyle, aroyle, hétéroaroyie ou hétérocyclique, R₁₀ représente un atome d'hydrogène, de fluor, de chlore, de brome ou d'iode ou un groupe alcynyle, alkyle, aroylé ou hétéroaroyle, et n vaut de 1 à 20, de préférence de 1 à 10 et en particulier de 1 à 5, les groupes latéraux de cess bases pouvant porter des groupes protecteurs.

3. Procédé conforme à l'une des revendications précédentes, **caractérisé en ce que** m vaut de 1 à 200 et de préférence de 1 à 100.

4. Procédé conforme à l'une des revendications précédentes, **caractérisé en ce que**, à chaque étape de réaction, on change au moins l'un des composants III, IV et/ou V.

5. Procédé conforme à l'une des revendications précédentes, **caractérisé en ce qu'**on le met en oeuvre sur une phase solide comme un support "chip".

6. Composé de formule 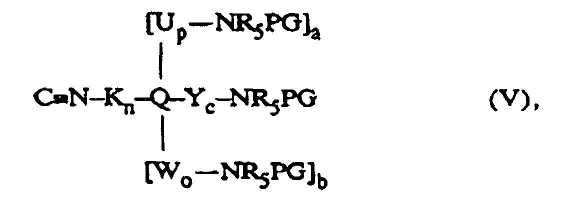 dans laquelle
les PG représentent, indépendamment les uns des autres, des groupes habituels de protection de fonctions amine, facilement clivables (protection temporaire), éventuellement orthogonaux, de la classe des dérivés N-silyle, ou des groupes protecteurs choisis parmi les suivants : tBoc, Alloc, Fmoc, Moz, Z, Tr, MMTr, DMTr, pixyle et TBDMS ;
les R₅ représentent, indépendamment les uns des autres, des atomes d'hydrogène, des groupes alkyle, cycloalkyle, alcoxyalkyle ou aryle, substitués ou non substitués, ou des groupes hétérocycliques ;
les U, W, K et Y représentent, indépendamment les uns des autres, des groupes alkyle, alcényle, alcynyle, alcanoyle, alcoxyalcanoyle, cycloalkyle ou aryle, substitués ou non substitués, des groupes hétérocycliques substitués ou non substitués, ou des groupes NR₅ où R₅ a la signification indiquée précédemment ;
a, b, c, n, o et p représentent chacun, indépendamment les uns des autres, un nombre entier qui vaut de 0 à 10, et de préférence de 0 à 5 ;
Q représente un groupe alkyle, aryle, alcényle, alcynyle ou cycloalkyle, substitué ou non substitué, un groupe hétérocyclique substitué ou non substitué, ou un groupe de formule SO₂ ;
tous les substituants mentionnés précédemment étant choisis dans l'ensemble suivant :
-OH, -Rₐ, -O-(cyclo)alkyle, -O-aryle, -O-hétéroaroyle, -O-hétérocyclique, -NH₂, -NO₂, -CN, -N₃, -CNRₐNR_{b}R_{c}, -NRₐR_{b}, -N⁺RₐR_{b}R_{c}, fluoro, chloro, bromo, ester d'acide (alpha, bêta, ... oméga)-aminé, -NRₐCOR_{b}, -NRₐCOXR_{b} (où X représente -O, -NR, -PO_{0,2,3,4}R, -SO_{0,1,2,4}R ou -NRₐNR_{b}R_{c}), -CORₐ, -COORₐ, -OCOORₐ, -CONRₐR_{b}, -OCONRₐR_{b}, -NR_{c}CONRₐR_{b}, -Rₐ-O-R_{b}, -R_{c}-NRₐR_{b}, -Rₐ-S-R_{b}, -Rₐ-SO-R_{b}, -Rₐ-SO₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, -NRₐCOR_{b}-O-R_{c}, -ORₐ-S-R_{b}, -NRₐR_{b}-S-R_{c}, -SO_{1,2,3,4}Rₐ-S-R_{b}, -CORₐ-S-R_{b}, -OCORₐ-S-R_{b}, -NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b} et -NR_{c}POORₐOR_{b}, où Rₐ, R_{b}, R_{c} et R_{d} peuvent représenter, indépendamment les uns des autres, des groupes (cyclo)alkyle, alcényle, alcynyle, aroyle, hétéroaroyle, hétérocycliques, aralkyle, aralcényle ou perhalogénoalkyle, tels que définis plus haut.

7. Composé conforme à la revendication 6, **caractérisé en ce que** :
les R₅ représentent, indépendamment les uns des autres, des atomes d'hydrogène ou des groupes alkyle ou aryle, substitués ou non substitués ;
les U, W, K et Y représentent, indépendamment les uns des autres, des groupes alkyle, cycloalkyle ou aryle, substitués ou non substitués ;
a, b, c, n, o et p représentent chacun, indépendamment les uns des autres, un nombre entier qui vaut de 0 à 5 ;
Q représente au moins un groupe alkyle, aroyle ou aryle, substitué ou non substitué, ou un groupe hétérocyclique substitué ou non substitué ;
tous les substituants mentionnés précédemment étant choisis dans l'ensemble suivant :
-OH, -Rₐ, -O-(cyclo)alkyle, -O-aryle, -O-hétéroaroyle, -O-hétérocyclique, -NH₂, -NO₂, -CN, -N₃, -CNRₐNR_{b}R_{c}, -NRₐR_{b}, -N⁺RₐR_{b}R_{c}, fluoro, chloro, bromo, ester d'acide (alpha, bêta, ... oméga)-aminé, -NRₐCOR_{b}, -NRₐCOXR_{b} (où X représente -O, -NR, -PO_{0,2,3,4}R, -SO_{0,1,2,4}R ou -NRₐNR_{b}R_{c}), -CORₐ, -COORₐ, -OCOORₐ, -CONRₐR_{b}, -OCONRₐR_{b}, -NR_{c}CONRₐR_{b}, -Rₐ-O-R_{b}, -R_{c}-NRₐR_{b}, -Rₐ-S-R_{b}, -Rₐ-SO-R_{b}, -Rₐ-SO₂-R_{b}, -ORₐ-O-R_{b}, -NRₐR_{b}-O-R_{c}, -SO₂Rₐ, -SO_{1,2,3,4}Rₐ-O-R_{b}, -CORₐ-OR_{b}, -COORₐ-O-R_{b}, -OCORₐ-O-R_{b}, -OCOORₐ-O-R_{b}, -NR_{b}CORₐ-O-R_{b}, -CONRₐR_{b}-O-R_{c}, -OCONRₐR_{b}-O-R_{c}, -NR_{c}CONRₐR_{b}-O-R_{d}, -NRₐCOR_{b}-O-R_{c}, -ORₐ-S-R_{b}, -NRₐR_{b}-S-R_{c}, -SO_{1,2,3,4}Rₐ-S-R_{b}, -CORₐ-S-R_{b}, -OCORₐ-S-R_{b}, ―NRₐCOR_{b}-S-R_{c}, -CONRₐR_{b}-S-R_{c}, -NRₐCONR_{b}R_{c}-S-R_{d}, -ORₐ-NR_{b}R_{c}, -NRₐR_{b}-NR_{c}R_{d}, -SO_{1,2,3,4}R_{b}-NR_{b}R_{c}, -CORₐ-NR_{b}R_{c}, -COORₐ-NR_{b}R_{c}, -OCORₐ-NR_{b}R_{c}, -OCOORₐ-NR_{b}R_{c}, -NRₐCONR_{b}R_{c}-NR_{d}, -NRₐCOOR_{b}-NR_{c}R_{d}, -OCONRₐR_{b}-NR_{c}R_{d}, -NRₐCONR_{b}R_{c}-NHR_{d}, -NRₐCOOR_{b}-NR_{c}R_{d}, -POORₐOR_{b} et -NR_{c}POORₐOR_{b}, où Rₐ, R_{b}, R_{c} et R_{d} peuvent représenter, indépendamment les uns des autres, des groupes (cyclo)alkyle, alcényle, alcynyle, aroyle, hétéroaroyle, hétérocycliques, aralkyle, aralcényle ou perhalogénoalkyle, tels que définis plus haut.

8. Composé conforme à la revendication 6 ou 7, choisi parmi ceux dont les structures suivent :

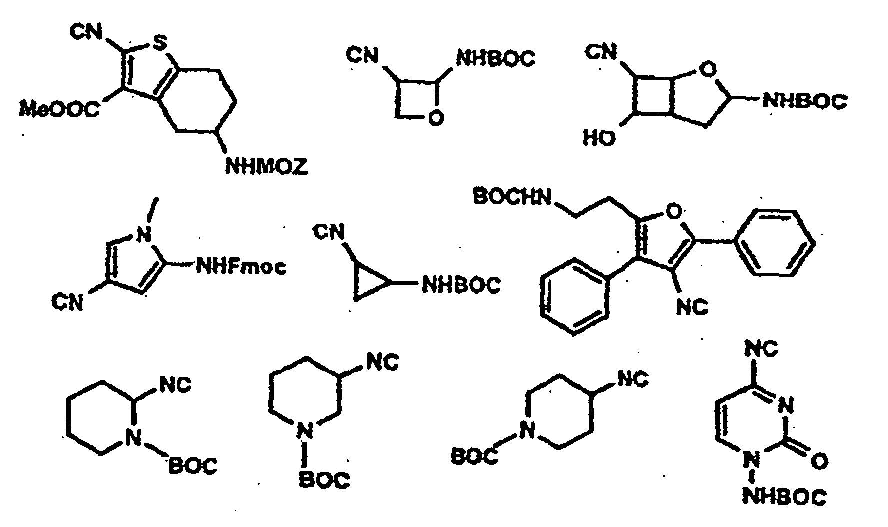

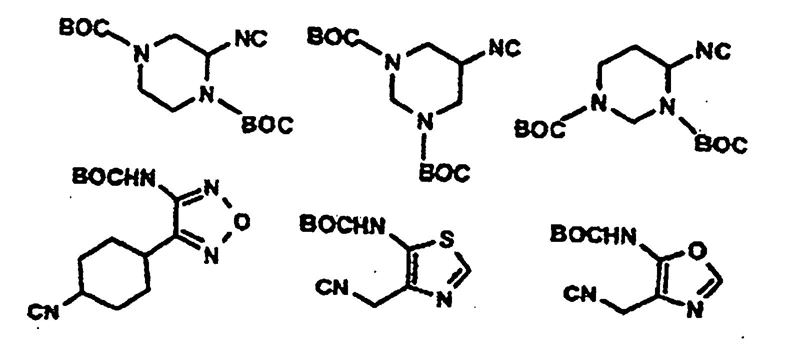

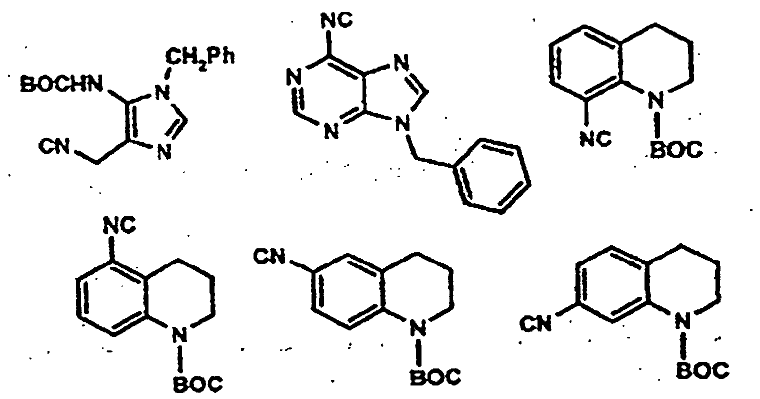

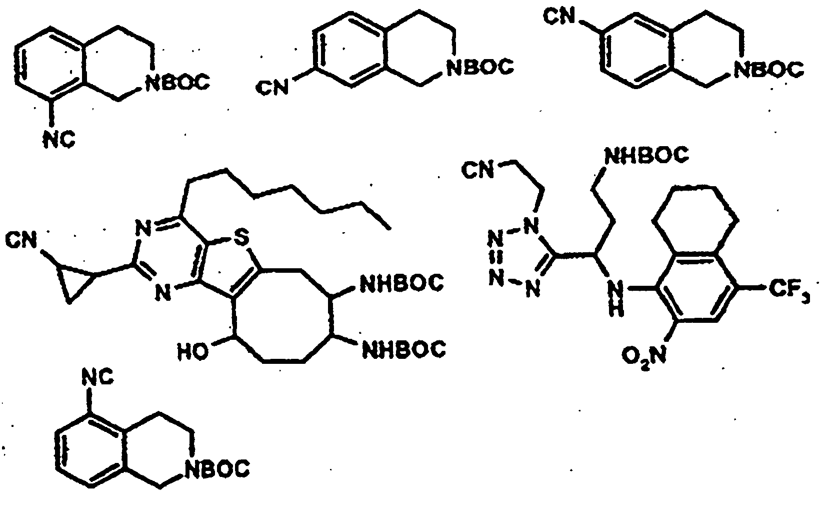

9. Procédé de préparation d'un composé de formule V, **caractérisé en ce qu'**on protège, en suivant les procédés habituels, un composé de formule VII : 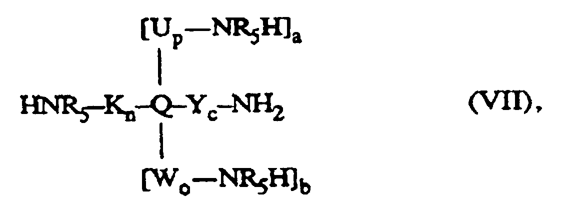 à l'aide de groupes PG définis dans la revendication 6, qui peuvent être indépendants les uns des autres, grâce à quoi l'on obtient un composé de formule VIII : 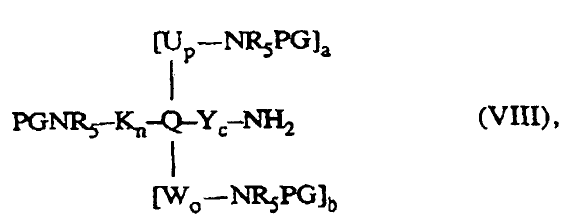 et l'on convertit ensuite le composé de formule VIII, en suivant les procédés habituels, en un isonitrile de formule V.

10. Procédé conforme à la revendication 9, **caractérisé en ce qu'**on convertit un composé de formule VIII, à l'aide d'un réactif habituel de formylation, en un composé de formule IX : 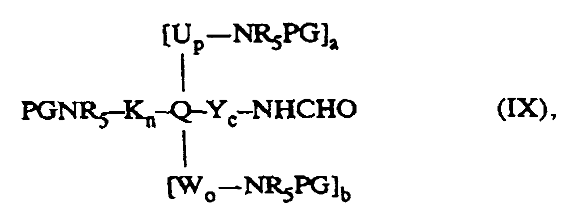 que l'on convertit ensuite en un composé de formule V en opérant dans les conditions habituelles, les groupes et les indices ayant les définitions indiquées dans la revendication 6.

11. Procédé de préparation d'un composé de formule IX, indiquée dans la revendication 10, **caractérisé en ce qu'**on part d'un composé de formule X : 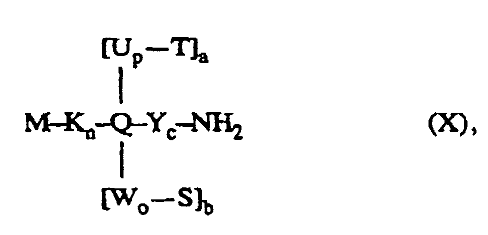 dans laquelle les groupes et les indices ont les définitions indiquées dans la revendication 6,
et **en ce que** l'on commence par effectuer la formylation de la fonction aminé, puis on transforme selon un procédé habituel un ou plusieurs des groupes fonctionnels M, T et S, qui représentent indépendamment les uns des autres des atomes d'halogène ou des groupes hydroxyle, en un ou plusieurs groupes fonctionnels azide, on convertit selon un procédé habituel ces azides en les amines correspondantes, et l'on munit celles-ci des groupes protecteurs indiqués plus haut, selon un procédé habituel.
